# EUROPÄISCHE PATENTSCHRIFT

(11) **EP 2 975 684 B1**
(45) Veröffentlichungstag und Bekanntmachung des Hinweises auf die Patenterteilung: **20.06.2018**
(21) Anmeldenummer: 15001775.4
(22) Anmeldetag: 29.06.2015
(51) Int. Cl.: H01M 10/0569, C07D 257/04, H01M 10/0525, H01M 10/0564

(54) **ELEKTROLYTSYSTEM FÜR DEN EINSATZ IN ELEKTROCHEMISCHEN BAUTEILEN**
ELECTROLYTE SYSTEM FOR USE IN ELECTROCHEMICAL COMPONENTS
SYSTEME D'ÉLECTROLYTE POUR L'UTILISATION DANS DES COMPOSANTS ÉLECTROCHIMIQUES

(30) Priorität: 18.07.2014 DE 102014010526
(43) Veröffentlichungstag der Anmeldung: 20.01.2016
(73) Patentinhaber: Forschungszentrum Jülich GmbH, 52425 Jülich (DE)
(72) Erfinder: Bergholz, Timm, 50226 Frechen (DE); Korte, Carsten, 52428 Jülich (DE); Sundermeyer, Jörg, 35041 Marburg (DE)

(56) Entgegenhaltungen:
- WO-A2-2006/078275
- CN-A- 103 814 468
- JP-A- 2004 331 521
- PATRIK JOHANSSON ET AL: "Novel Hückel stabilised azole ring-based lithium salts studied by ab initio Gaussian-3 theory", PHYSICAL CHEMISTRY CHEMICAL PHYSICS., Bd. 6, Nr. 5, 5. Februar 2004 (2004-02-05) , Seite 895, XP055227028, GB ISSN: 1463-9076, DOI: 10.1039/b313684a
- FINNEGAN W G ET AL: "An improved synthesis of 5-substituted tetrazoles", JOURNAL OF THE AMERICAN CHEMICAL SOCIETY, AMERICAN CHEMICAL SOCIETY, US, Bd. 80, 1. August 1958 (1958-08-01), Seiten 3908-3911, XP002317817, ISSN: 0002-7863, DOI: 10.1021/JA01548A028
- EARNEST OBED JOHN ET AL: "5-(Perfluoroalkyl)tetrazoles: .eta.5 ligands in solution and .mu.-2,3-.eta.2 ligands in solid complexes", INORGANIC CHEMISTRY, Bd. 28, Nr. 5, 1. März 1989 (1989-03-01), Seiten 893-897, XP055226852, EASTON, US ISSN: 0020-1669, DOI: 10.1021/ic00304a018
- EARNEST OBED JOHN ET AL: "Reactions of 5-(perfluoroalkyl)tetrazolates with cyanogen, nitrosyl, and cyanuric chlorides", INORGANIC CHEMISTRY, Bd. 28, Nr. 26, 1. Dezember 1989 (1989-12-01), Seiten 4629-4633, XP055226862, EASTON, US ISSN: 0020-1669, DOI: 10.1021/ic00325a018
- CRAWFORD M J ET AL: "Synthesis and characterization of perfluorinated nitriles and the corresponding sodium 5-perfluoroalkyltetrazolate salts", JOURNAL OF FLUORINE CHEMISTRY, ELSEVIER, NL, vol. 129, no. 12, 1 December 2008 (2008-12-01), pages 1199-1205, XP025678338, ISSN: 0022-1139, DOI: 10.1016/J.JFLUCHEM.2008.09.007 [retrieved on 2008-09-25]

## Beschreibung

Die Erfindung betrifft ionische Flüssigkeiten und Salze zur Verwendung in Elektrolytsystemen zur lonenleitung und Optimierung im Rahmen der Sicherheit, der Leistung und der Lebensdauer von elektrochemischen Bauteilen. Die Erfindung betrifft insbesondere die Optimierung eines Elektrolytsystems für Lithium-basierte Energiespeicher (engl.: Lithium-Ion Battery, LiB), insbesondere solche Lithium-basierte Energiespeicher, die Kathoden mit hohen Potentialen gegenüber Lithium enthalten.

### Stand der Technik

Die Vorteile von Lithiumionenbatterien (LiBs) gegenüber anderen Akkumulatoren mit wässrigen Elektrolyten (beispielsweise Blei- oder Nickelmetallhydrid-Batterien) sind eine höhere Energie- und Leistungsdichte aufgrund der hohen Zellspannung, die lange Lagerfähigkeit durch eine geringe Selbstentladung, der leicht erweiterte Temperaturbereich für den Betrieb sowie eine nicht erforderliche Wartung.

Das grundlegende Prinzip einer Lithiumionenbatterie entspricht weitgehend dem der übrigen Akkumulatoren, wie beispielsweise eines Pb-, NiCd- oder NiMH-Akkumulators. In einer Lithiumionenbatterie sind zwei Elektroden, Anode und Kathode, über den Elektrolyten ionisch und über den Verbraucher elektrisch miteinander verbunden. Der Elektrolyt ist ein elektronischer Isolator und ein ionischer Leiter, der die Elektroden ohne geschlossenen elektronischen äußeren Stromkreis elektrisch und mechanisch voneinander isoliert. In der Regel werden Flüssigelektrolyte eingesetzt. In diesem Fall sorgt ein fester Separator zwischen den Elektroden für die mechanische Separierung. Der Elektrolyt umfasst typischerweise ein Salz von Lithium, gelöst in einem oder mehreren, typischerweise nicht-wässrigen (aprotischen), organischen Lösungsmitteln.

Die Elektroden sind elektrochemisch aktive Materialien, die unterschiedliche elektrochemische Potentiale aufweisen. Die Elektrode mit dem geringeren Potential wird als Minuspol bezeichnet, die mit dem höheren als Pluspol. Entsprechend der Reaktionsführung bei der Entladung werden die beiden Elektroden als Batterieanode und Batteriekathode bezeichnet. Die Batteriespannung ergibt sich aus ihrem Potentialunterschied. Wenn der äußere Stromkreis über die Anoden- (Cu) und Kathodenstromkollektoren (Al) geschlossen wird, tritt die elektrische Kontaktierung ein. In der Folge diffundieren Li⁺-Ionen bei der Entladung durch den Elektrolyten von der Anode (Minuspol) zur Kathode (Pluspol). Der Transport von Li⁺ durch den Separator erfolgt nach Solvatisierung durch die Elektrolytmoleküle. Bei der Entladung läuft dieser Prozess freiwillig ab. Bei der Ladung wird die Stromrichtung geändert und der Fluss von Li⁺-Ionen erfolgt von der Kathode zur Anode.

Die Steigerung des Kathodenpotentials in Hochvoltkathoden in neuen Lithiumbatterie-Konzepten führt zu erhöhten Energie- und Leistungsdichten.

Die derzeitig eingesetzten Elektrolytsysteme in Lithiumbatterien bestehen in der Regel aus organischen Lösungsmitteln, vor allem aus Mischungen von azyklischen (z. B. Dimethylcarbonat (DMC), Diethylcarbonat (DEC) oder Ethylmethylcarbonat (EMC)) und zyklischen Carbonaten (z. B. Ethylencarbonat (EC) oder Propylencarbonat (PC)), die das Lösungsmittel für das Lithiumsalz als Leitsalz darstellen. Dieses besteht üblicherweise aus Lithiumhexafluorophosphat (LiPF₆) oder Lithiumtetrafluoroborat (LiBF₄), die in den organischen Lösungsmitteln größtenteils dissoziiert vorliegen.

Die Vorteile dieser Mischungen sind eine gute Löslichkeit des Leitsalzes in Verbindung mit einer hohen Lithiumionenleitfähigkeit, keine Korrosion der Aluminiumstromkollektoren und die Formierung einer festen Elektrolytgrenzfläche (engl.: solid electrolyte interface, SEI) auf Graphit. Die Bildung der festen Elektrolytgrenzfläche verhindert weitgehend die fortlaufende Reduktion des Lösungsmittels während der Zyklisierung.

Folgende Nachteile derzeitiger Elektrolytsysteme schränken die Eigenschaften von Lithiumbatterien ein ^{[1]}:
- eine mangelnde elektrochemische und thermische Stabilität,
- die Brennbarkeit und Flüchtigkeit der Lösungsmittel,
- die geringen Starttemperaturen der thermischen Reaktion des Elektrolyten mit den Elektroden unter Bildung von flüchtigen Reaktionsprodukten,
- die Hydrolyse durch die Reaktion des Lithiumsalzes mit protischen Verunreinigungen, die im ppm-Konzentrationen immer in den Akkumulatoren vorhanden sind, zu HF,
- relativ hohe Schmelztemperaturen von mehr als -10 bis -20 °C, die die Anwendung bei geringen Temperaturen nur bedingt ermöglichen; das in der Regel eingesetzte Ethylencarbonat (EC) führt zu einer minimalen Anwendungstemperatur von mehr als -10 °C,
- eine ausreichende elektrochemische Stabilität gegenüber dem Kathoden- und Anodenpotential ist vor allem für Graphitanoden und Hochvoltkathoden nicht gegeben,

Diese Nachteile führen regelmäßig zu Lebensdauer- und Sicherheitsproblemen derzeitiger Lithiumionenbatterien. Der hohe Dampfdruck derzeitiger Elektrolyte kann zu einem Aufblähen oder Explodieren der Zellen führen. Die Brennbarkeit konventioneller organischer Lösungsmittel in Lithiumionenbatterien oder Doppelschichtkondensatoren führt zu möglichen Feuern und Explosionen der Bauteile. Die geringe thermische Stabilität konventioneller organischer Lösungsmittel gegenüber Lithiumbatterieelektroden führt zudem zu hohen frei werdenden Reaktionsenthalpien beim thermischen Durchgehen (thermal runaway) der Zellen.

Ein erster Lösungsansatz besteht im Einsatz von so genannten ionischen Flüssigkeiten als Lösungsmittel. Dabei werden häufig hydrophobe ionische Flüssigkeiten, wie z. B. die Verbindungen Ethylmethylimidazoliumbis(trifluormethylsulfonyl)imid [EtMelm][TFSI] oder N,N-Butylmethylpyrrolidiniumbis(trifluormethylsulfonyl)imid [BuMePy][TFSI] verwendet.

Außerdem wird der Einsatz von stabilen Li-Salzen diskutiert, beispielsweise in DE 19829030 C1 die Lithiumsalze des Bisoxalatoborats (LiBOB).

Weiterhin wurden Bis(trifluormethylsulfonyl)imide (LiTFSI), Lithiumbis(fluorsulfonyl)imide (LiFSI) sowie Mischungen von herkömmlichen und den vorgenannten Salzen, z. B. LiPF₆ und LiBOB in diesem Zusammenhang erforscht.

Es hat sich herausgestellt, dass Salze, vor allem von ionischen Flüssigkeiten mit Azolbasierten Anionen, generell interessante Eigenschaften als Elektrolytkomponenten aufweisen.

Die Vorteile von Elektrolyten aus ionischen Flüssigkeiten und den vorgenannten Lithiumsalzen bestehen darin, dass sie regelmäßig:
- einen vernachlässigbaren Dampfdruck aufweisen,
- eine hohe Zündtemperatur und hohe Flammpunkte (T_{F} > 200 °C) aufweisen,
- einen breiten Flüssigkeitsbereich aufzeigen,
- eine hohe chemische Stabilität gegenüber O₂, H₂O und Li aufweisen,
- eine hohe elektrochemische Stabilität (z. B. ΔU([EtMelm][TFSI], Pt) = 4,3 V)^{[1]}
- und eine hohe thermische Stabilität (z. B. T_{Dekomp}([EtMelm][TFSI]) = 400 °C)^{[1]} zeigen,
- bei der thermischen Reaktion mit den Elektroden im Vergleich zu organischen Lösungsmittelelektrolyten weniger gasförmige Produkte bilden ^{[2]}.

Weiterhin sind die Eigenschaften der Ionen (Brönstedacidität, Lewisacidität und die Hydrophilie) vorteilhaft durch chemische Modifikation in breiten Bereichen einstellbar.
Bislang wurden aber für die Elektrolytsysteme aus ionischen Flüssigkeiten und den vorgenannten Lithiumsalzen immer auch einige Nachteile bzw. Probleme beobachtet:
- Die ionische Flüssigkeit-Salzmischung weist häufig eine hohe Viskosität auf;
- Die Gesamtionenleitfähigkeit im Vergleich zu organischen Lösungsmittelelektrolyten ist geringer;
- Die Löslichkeit des Lithiumleitsalzes beträgt häufig weniger als 0,1 mol/kg, und ist damit sehr gering, was in Folge zu einer geringen Lithiumtransportzahl t_{Li} und ebenfalls als Folge zu geringen Lithiumionenleitfähigkeiten führt;
- Es findet keine Ausbildung einer festen Elektrolytgrenzfläche (SEI) auf Graphitanoden statt, wodurch Cointerkalation von Kationen der ionischen Flüssigkeiten und damit einhergehend Delamination der Anode auftritt;
- Einige der bekannten ionischen Flüssigkeiten weisen eine mangelnde Stabilität gegenüber der Reduktion an Lithium- oder Graphitanode auf;
- Einige der bekannten ionischen Flüssigkeiten weisen eine mangelnde Stabilität gegenüber Hochvoltkathoden bei Potentialen gegen Lithium von mehr als 5 V auf.
So wird beispielsweise in CN 103814468 A ein Elektrolyt für Lithiumbatterien offenbart, der eine ionische Flüssigkeit und ein Lithiumsalz enthält. Die Zusammensetzung der ionischen Flüssigkeit wird als N-Isopropylpyridinium 5-Nitrotetrazolid angegeben
In W. Finnegan et al. "An improved synthesis of 5-substituted tetrazoles", Journal of the American Chemical Society, US, Bd. 80, 1. August 1958 (1958-08-01), Seiten 3908-3911, XP002317817, ISSN: 0002-7863, DOI: 10.1021/JA01548A028 ist eine ionische Flüssigkeit umfassend das Kaliumsalz eines Tetrazolidanions A mit einem Substituenten R = C₇F₁₅ offenbart.
In E.O.John et al.: "5-(Perfluoroalkyl)tetrazoles: η5 Ligands in Solution and µ-2,3-η2 Ligands in solid complexes", Inorganic Chemistry, Bd. 28, Nr. 5, 1. März 1989 (1989-03-01), Seiten 893-897, XP055226852, Easton, US, ISSN: 0020-1669,001: 10.1021/ic00304a018 wird ein Verfahren zur Herstellung eines Tetrazolidanions A mit einem Substituenten R = CF₃ in dem organischen Lösungsmittel Acetonitril beschrieben, zwischen einem ionischen Azid, welches Na⁺ umfasst, und einer durch den Rest R = CF₃ substituierten Cyanogruppe R-CN.
In E. O. John et al.: "Reactions of 5-(perfluoroalkyl)tetrazolates with cyanogen, nitrosyl, and cyanuric chlorides", Inorganic Chemistry, Bd. 28, Nr. 26, 1. Dezember 1989 (1989-12-01), Seiten 4629-4633, XP055226862, Easton, US, ISSN: 0020-1669,001: 10.1021/Ic00325a018 wird zudem das Natriumsalz des Tetrazolidanions A mit den Substituenten R = CF₃ und R = C₂F₅ offenbart.
Aus M. Crawford et al.: "Synthesis and characterization of perfluorinated nitriles and the corresponding sodium 5perfluoroalkyltetrazolate salts", Journal of Fluorine Chemistry, Elsevier, NL, Bd. 129, Nr. 12, 1. Dezember 2008 (2008-12-01), Seiten 1199-1205, XP025678338, ISSN: 0022-1139,001: 10.1016/J.JFLUCHEM.2008.09.007 sind Natriumsalze des Tetrazolidanions A mit dem Substituent R = -(CF₂)ₙCF₃, für n = 0, 1 und 2 und ihre Herstellung nach der in Anspruch 6 beschriebenen Methode bekannt.
WO 2006/078275 A2 offenbart ionische Flüssigkeiten, die auf einem Tetrazolidanion basieren und insbesondere ein Tetrazolidanion A mit R =OR³², Ethergruppe, wobei R³² eine mit den elektronenziehenden Substituenten Fluor oder Azid substituierte Alkylgruppe ist,
Auch aus JP 2004 331521 A sind ionische Flüssigkeiten mit hoher ionischer Leitfähigkeit bekannt, wobei die ionische Flüssigkeit durch die Formel K⁺A⁻ representiert wird, mit K⁺ = N-Alkylimidazolkation oder Ammoniumkation und A- = Tetazolidanion oder Triazolidanion.
Kein bekannter Elektrolyt umfassend ionische Flüssigkeiten kombiniert bislang alle Vorteile, ohne auch entsprechende Nachteile mit sich zu bringen. Problematisch ist dabei, dass die verschiedenen Faktoren nicht gleichförmig mit einer Veränderung der chemischen Struktur von Anion oder Kation beeinflusst werden.
Die angesprochenen Probleme der bisher bekannten Elektrolyte führen in der Regel zu einer geringen Lebensdauer. Insbesondere führen sie zu einer hohen Rate der Kapazitäts- und Leistungsabnahme. Letztere durch eine Erhöhung des Widerstandes. Außerdem ist die Leistungsdichte von LiBs mit Elektrolyten auf Basis von ionischen Flüssigkeiten aufgrund ihrer geringen Lithiumionenleitfähigkeit gering.

### Aufgabe und Lösung

Die Aufgabe der Erfindung ist es, einen gegenüber dem Stand der Technik verbesserten Elektrolyten für ein elektrochemisches Bauteil bereit zu stellen, welcher eine deutlich verbesserte lonenleitung aufweist, thermisch und chemisch stabil ist, und insbesondere für den Einsatz in Lithium-basierten Energiespeichern geeignet ist. Ferner soll der verbesserte Elektrolyt eine hohe Leitfähigkeit, eine geringe Viskosität und einen breiten Flüssigkeitsbereich aufweisen. Die Anwendung der Bauteile erfolgt typischerweise in Temperaturbereichen von -30 °C bis 80 °C.

Die Aufgabe der Erfindung wird gelöst durch einen Elektrolyten umfassend Tetrazolidbasierte Anionen als Lösungsmittel, Leitsalz und/oder Additiv für den Einsatz in einem elektrochemischen Bauteil gemäß Hauptanspruch.

Ferner wird die Aufgabe der Erfindung gelöst durch die Bereitstellung neuer Tetrazolidbasierter Anionen gemäß erstem Nebenanspruch, sowie durch Verfahren zu deren Herstellung gemäß weiterer Nebenansprüche.

Vorteilhafte Ausgestaltungen des Elektrolyten sowie der Tetrazolid-basierten Anionen finden sich in den jeweils darauf rückbezogenen Ansprüchen.

### Gegenstand der Erfindung

Die Erfindung betrifft ionische Flüssigkeiten und Salze zur Verwendung in Elektrolytsystemen in elektrochemischen Bauteilen. Die erfindungsgemäßen Substanzen dienen dabei:
- als Elektrolyt, der die lonenleitung in elektrochemischen Bauteilen (Batterien, Kondensatoren, Brennstoffzellen) realisiert,
- als Lösungsmittel des Elektrolyten, zur Solvatisierung eines Leitsalzes,
- als Leitsalz des Elektrolyten, der den ionischen Transport gewährleistet,
- als Additiv im Elektrolyten, das die Eigenschaften (Sicherheit, Alterung, Leistung) des elektrochemischen Bauteils optimiert.

In herkömmlichen Elektrolyten für elektrochemische Bauteile, insbesondere für Lithiumakkumulatoren, ist in der Regel die Leitfähigkeit in Verbindung mit einem ausreichenden elektrochemischen und thermischen Stabilitätsfenster nicht groß genug oder es werden keine ausreichenden stabilisierenden Oberflächenschichten außerhalb des elektrochemischen Stabilitätsfensters gebildet. Weiterhin ist die Leitfähigkeit bei geringen Temperaturen häufig nicht ausreichend.

Die Erfindung beschreibt die Darstellung und Eigenschaften solcher Systeme, die die Nachteile des bisherigen Standes der Technik überwinden.

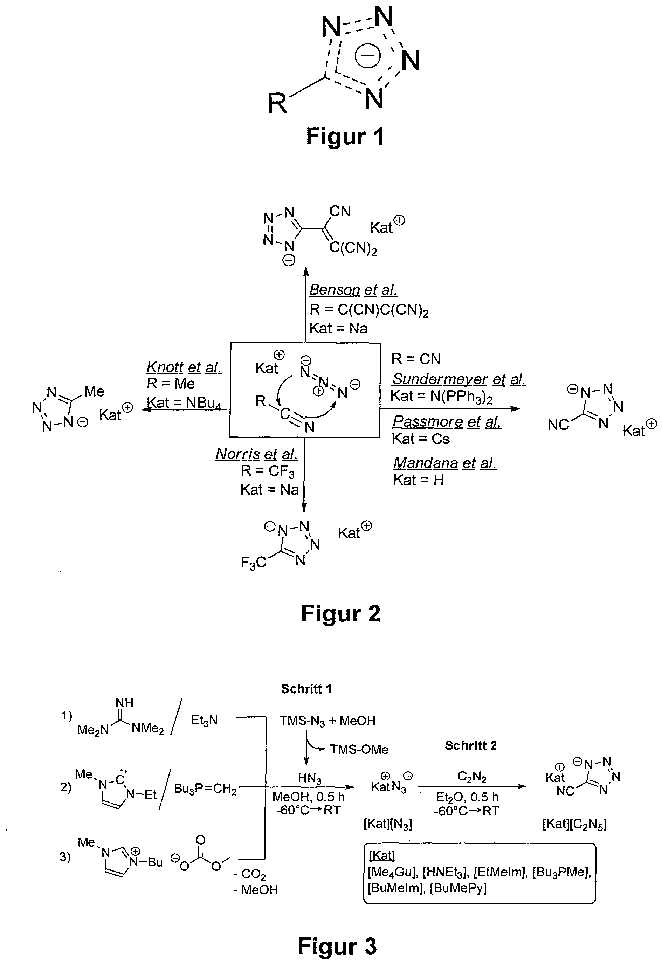

Durch die Einführung von neuen Tetrazolid-basierten Anionen, die in 5-Position substituiert sind (siehe Figur 1), sowie durch bekannte Tetrazolidanionen, die in 5-Position substituiert sind, in Kombination mit geeigneten Kationen werden im Rahmen der Erfindung neue ionische Flüssigkeiten und Salzverbindungen bereit gestellt, die vorteilhaft als Lösungsmittel für die lonenleitung in elektrochemischen Bauteilen und dabei insbesondere in Lithiumbatterien eingesetzt werden können. Die an der 5-Position substituierten Reste zeichnen sich alle durch eine elektronenziehende Wirkung aus, die die negative Ladung des Tetrazolrings durch einen induktiven und/oder mesomeren Effekt stabilisiert.

Unter einer ionischen Flüssigkeit (engl.: ionic liquid, IL) wird eine Verbindung verstanden, die ausschließlich aus Ionen besteht, einen Schmelzpunkt unterhalb von 100 °C und einen vernachlässigbaren Dampfdruck aufweist. Vertreter, die bereits bei Raumtemperatur flüssig vorliegen, werden als Raumtemperatur-ionische Flüssigkeiten (engl.: room temperature ionic liquids, RT-ILs) bezeichnet.

Demgegenüber wird von Salzen gesprochen, wenn die ionischen Verbindungen einen Schmelzpunkt oberhalb von 100 °C aufweisen.

Generell bestehen die im Rahmen dieser Erfindung bereitgestellten Verbindungen aus verschiedenen Tetrazolid-basierten Anionen und bekannten Kationen. Diese können einzeln oder auch als Mischungen mit herkömmlichen Salzen, weiteren Additiven, herkömmlichen Lösungsmitteln, Polymeren oder herkömmlichen ionischen Flüssigkeiten vorteilhaft als Elektrolytsystem in elektrochemischen Bauteilen eingesetzt werden.

Ein Elektrolytsystem im Rahmen der Erfindung umfasst wenigstens eine chemische Verbindung (ionische Flüssigkeit oder Salz), die im festen, flüssigen oder gelöstem Zustand in Ionen (Anionen und Kationen) dissoziiert vorliegt, wobei sich die Ionen unter dem Einfluss eines elektrischen Feldes entsprechend bewegen können. Das Elektrolytsystem umfasst zudem wenigstens eine erfindungsgemäße Verbindung aus einem Tetrazolid-basierten Anionen und einem Kation.

Im Rahmen der Erfindung werden unter elektrochemischen Bauteilen Brennstoffzellen, Elektrolysezellen und Batterien (Akkumulatoren), und dabei insbesondere Lithiumakkumulatoren verstanden. Auch Dual-Ion-Batterien, Redoxflusszellen, Metallluftbatterien, Metallhydridbatterien oder abgeleitete Zellkonzepte können im Rahmen der Erfindung darunter verstanden werden.

### 1. Erfindungsgemäße ionische Flüssigkeiten oder Salze

Die im Rahmen der Erfindung eingesetzten Tetrazolidanionen (A2 bis A10; A1 ist nicht erfindungsgemäß und dient nur als Referenz) sind schematisch mit ihrer Strukturformel der Tabelle 1 zu entnehmen. Sie zeichnen sich durch unterschiedliche elektronenziehende Substituenten in 5-Position aus, die vorteilhaft die negative Ladung durch einen induktiven und oder mesomeren Effekt stabilisieren.
Es können insbesondere folgende Substituenten R eingesetzt werden, die die Tetrazolidanionen A1 bis A10 kennzeichnen (der Index n steht für die Anzahl der C-Atome in den Substituenten):
- R = Cyanogruppe (-CN), (A1; nicht erfindungsgemäß; dient nur als Referenz)
- R = Fluorgruppe (-F), (A2)
- R = Trifluormethylgruppe (-CF₃), (A3)
- R = Perfluoralkylgruppe (R_{f}), mit R_{f} = -(CF₂)ₙ-CF₃ und n = 1, 2, 3, 4, 5, 6, 7. (A4ₙ)
- R = Fluorsulfonylgruppe (-SO₂F), (A5)
- R= Trifluormethylsulfonylgruppe (-SO₂CF₃), (A6)
- R = Sulfonylgruppe mit höheren fluorierten Akylrest, Perfluoralkylsulfonlygruppe (-SO₂R_{f}), mit R_{f} = -(CF₂)ₙ-CF₃ und n = 1, 2, 3, 4, 5, 6, 7. (A7ₙ)
- R = Perfluoralkylcarboxylgruppe (-OCOR_{f})
   mit R_{f} = -(CF₂)ₙ-CF₃ und n = 0, 1, 2, 3, 4, 5, 6, 7, (A8ₙ)
- R = Perfluoralkoxygruppe (-OR_{f})
   mit R_{f} = -(CF₂)ₙ-CF₃ und n = 0, 1, 2, 3, 4, 5, 6, 7, (A9ₙ)
- R = Cyanosulfonylgruppe (-SO₂CN) (A10)
Der perfluorierte Rest R_{f} in -R_{f}, -SO₂-R_{f}, -O-CO-R_{f} oder -O-R_{f} besteht beispielsweise aus verschiedenen linearen, vollständig fluorierten Alkylgruppen des allgemeinen Aufbaus - (CF₂)ₙ-CF₃, wobei n Werte von eins bis sieben annehmen kann, d. h. von einer Pentafluorethylgruppe bis zu einer Perfluorooctylgruppe.
Ferner kann R_{f} eine perfluorierte Isopropylgruppe (CF-(CF₃)₂) oder andere verzweigte perfluorierte Alkylgruppen umfassen.
Weiterhin ist die Einführung von Fluoroetherresten des allgemeinen Aufbaus: -O-(CF₂)ₙ-CF₃, -(O-CF₂CF₂)ₘ-CF₃, -(CF₂)m-O-(CF₂)ₙ-CF3, -O-CFCF₃CF₂-CF₃, -(CF₂)ₘ-O-CFCF₃CF₂-CF₃ für n = 1, 2, 3, 4 oder 5 und m = 1, 2, 3, 4 oder 5 möglich.

Während die Tetrazolidanionen A1, A2, A3 und A4ₙ für n = 1 und 2 bereits bekannt sind, wurden die übrigen in Tabelle 1 aufgeführten Tetrazolidanionen A4ₙ mit n = 3 bis 7, A5, A6, A7ₙ mit n = 1 bis 7, A8ₙ mit n = 0 bis 7 und A9ₙ mit n = 1 bis 7 sowie A10 als neue Substanzklasse im Rahmen der Erfindung bereitgestellt.

Dabei handelt es sich um ein Tetrazolidanion A mit einem Substituenten R in 5-Position wobei der Substituent R eine Perfluoralkylgruppe oder eine durch einen Elektronenziehenden Substituenten substituierte Sulfonylgruppe oder eine durch einen Elektronenziehenden Substituenten Carboxylgruppe oder eine durch einen Elektronen-ziehenden Substituenten substituierte Ethergruppe umfasst.

Die neue Substanzklasse zeichnet sich durch eine Substituierung des Tetrazolidanions in 5-Position durch eine Fluorsulfonylgruppe, eine Trifuormethylsulfonylgruppe, eine Perfluoralkylsulfonylgruppe, eine Perfluoralkylcarboxylgruppe, einer Cyanosulfonylgruppe oder einer Perfluoralkylethergruppe aus. Die perfluorierten Alkylsubstituenten können dabei linear oder verzweigt sein und Kettenlängen von n = 1 bis 8 aufweisen. Höhere Kettenlängen würden zu geringeren Mobilitäten der Anionen, höheren Schmelzpunkten und damit verbunden geringeren Leitfähigkeiten und höheren Viskositäten führen.

Wie beispielsweise anhand der Kristallstrukturdaten von [EtMelm][A1] belegt werden kann, ist die negative Ladung des Tetrazolidanions im planar vorliegenden 6π-System des Tetrazolidrings delokalisiert. Das Anion kann somit den Hückelaromaten zugeordnet werden und weist eine hohe thermische und elektrochemische Stabilität auf.

Die Eigenschaften der resultierenden Salze bzw. ionischen Flüssigkeiten variieren in Abhängigkeit der Substituenten des Tetrazolidanions.

Die erfindungsgemäßen ionischen Flüssigkeiten, also Salze, die bei unter 100 °C flüssig vorliegen, auf Basis der verschiedenen Tetrazolidanionen A1 bis A10 werden mit folgenden Kationen K1 bis K8, die eine geringe Ladungsdichte aufweisen, erhalten:
- Ammoniumkationen mit verschiedenen Resten (NRR¹R2R³),
   mit R, R¹, R², R³ = -(CH₂)ₙ-CH₃ und n = 0, 1, 2, 3, 4, 5, 6, 7. Die Reste R, R¹ R², R³ kön= nen dabei unterschiedlich oder auch gleich sein. (K1)
   - Imidazoliumkationen mit verschiedenen Resten in 1-, 2-, 3-, 4- und 5-Position (RR¹R²R³R⁴Im), mit R, R¹, R², R³, R⁴ = H, -(CH₂)ₙ-CH₃ und n = 0, 1, 2, 3, 4, 5, 6, 7. Die Reste R, R¹, R², R³, R⁴ können dabei unterschiedlich oder auch gleich sein. (K2)
   - Pyrrolidiniumkationen mit verschiedenen Resten in 1-Position (RR¹Py), mit R, R¹ = -(CH₂)ₙ-CH₃ und n = 0, 1, 2, 3, 4, 5, 6, 7. Die Reste R, R¹ können dabei unterschiedlich oder auch gleich sein. (K3)
   - Piperidiniumkationen mit verschiedenen Resten in 1-Position (RR¹Pip), mit R, R¹ = -(CH₂)ₙ-CH₃ und n = 0, 1, 2, 3, 4, 5, 6, 7.Die Reste R, R¹ können dabei unterschiedlich oder auch gleich sein. (K4)
   - Pyridiniumkationen mit verschiedenen Resten in 1-Position (RPyri), mit R = -(CH₂)ₙ-CH₃ und n = 0, 1, 2, 3, 4, 5, 6, 7. (K5)
   - Pyrazoliumkationen mit verschiedenen Resten in 1-, 2-, 3-, 4- und 5-Position (RR¹R²R³R⁴Pyr), mit R, R¹, R², R³, R⁴ = H, -(CH₂)ₙ-CH₃ und n = 0, 1, 2, 3, 4, 5, 6, 7. Die Reste R, R¹, R², R³, R⁴ können dabei unterschiedlich oder auch gleich sein. (K6)
   - Phosphoniumkationen mit verschiedenen Resten (PRR¹R²R³), mit R, R¹, R², R³ = - (CH₂)ₙ-CH₃ und n = 0, 1, 2, 3, 4, 5, 6, 7. Die Reste R, R¹, R², R³ können dabei unterschiedlich oder auch gleich sein. (K7)
   - Sulfoniumkationen mit verschiedenen Resten (SRR¹R²), mit R, R¹, R² = -(CH₂)ₙ-CH₃ und n = 0, 1, 2, 3, 4, 5, 6, 7. Die Reste R, R¹, R², R³ können dabei unterschiedlich oder auch gleich sein. (K8)
Je nach Charakter des Kations werden auch ionische Flüssigkeiten generiert, die nicht nur bei unter 100 °C, sondern darüber hinaus bereits bei Raumtemperatur (RT) flüssig vorliegen und daher in der Literatur RT-ionische Flüssigkeiten genannt werden.

So weist beispielsweise die nicht erfindungsgemäße ionische Flüssigkeit mit A1-Anion und K2-Kation mit Butyl- und Methylrest in 1- und 3-Position (Butylmethylimidazolium-5-cyano-tetrazolid, [BuMelm][A1]) einen Schmelzpunkt von T_{Smp}= 4 °C auf.

Ferner weist die nicht erfindungsgemäße ionische Flüssigkeit mit A1-Anion und K3-Kation mit Butyl- und Methylrest in 1-Position (Butylmethylpyrrolidinium-5-cyanotetrazolid, [BuMe-Py][A1]) einen Schmelzpunkt von T_{Smp}= -13 °C auf.

Mit den vorgenannten Tetrazolidanionen A2 bis A10 (erfindungsgemäß) und A1 (nicht erfindungsgemäß) können darüber hinaus mit den nachfolgend aufgeführten Kationen S1 bis S10, die jeweils eine hohe Ladungsdichte aufweisen, auch Salze hergestellt werden, die erst deutlich oberhalb von 100 °C schmelzen:
- H⁺, (S1)
- Li⁺, (S2)
- Na⁺, (S3)
- K⁺, (S4)
- Mg²⁺, (S5)
- Ca²⁺, (S6)
- Ni²⁺, (S7)
- Pb²⁺ (S8)
- Fe^{2+/3+}, (S9)
- V^{2+/3+/4+/5+}. S(10)
Für den Einsatz in einer Lithiumbatterie (LiB) sind dementsprechend die erfindungsgemäßen Salze auf Basis von S2 als Leitsalz und oder als Additiv besonders interessant.
Die vorgenannten ionischen Flüssigkeiten und Salze können vorteilhaft als Lösungsmittel, Leitsalze und Additive für die lonenleitung in elektrochemischen Bauteilen und dabei insbesondere in Batterien eingesetzt werden.

### 2. Syntheserouten für die neuen ionischen Flüssigkeiten und Salze

Die Synthese der freien Säure des 5-Cyanotetrazols erfolgte erstmals 1913 durch Mandana *et al.* Diese setzten HN₃ mit Dicyan um und konnten das Natrium-5-cyanotetrazolid durch Deprotonierung mit NaOH erhalten. Sundermeyer et al. gelang es 1990, das 5-Cyanotetrazolid als Bis(triphenylphosphoranyliden)iminiumsalz direkt durch Umsetzung des ionischen Azids mit Dicyan zu generieren, wie in Figur 2 schematisch dargestellt ist ^{[3]}.
Es wurde herausgefunden, dass in Analogie zur Umsetzung von Sundermeyer et al. alle übrigen Salze der Anionen gemäß der Tabelle 1 synthetisiert werden können, indem der Rest R in Figur 2 entsprechend verändert wird.

Die Darstellung der vorgenannten verschiedenen ionischen Flüssigkeiten mit einem A1-Anion ist beispielsweise aus der Literatur bekannt. In dieser Arbeit wird eine kostengünstige, hochskalierbare Syntheseroute angegeben, die analog auch für die im Rahmen dieser Erfindung bereitgestellten Tetrazolidanionen eingesetzt werden kann.

### a. Syntheseweg für Anion A1

Ionische Flüssigkeiten mit Anion A1) können gemäß des Reaktionsschemas in Figur 3 - Schritt 2 in einer [2+3]-Cycloadditionsreaktion zwischen den ionischen Aziden mit Dicyan (C₂N₅) dargestellt werden.

Die Azide werden in z. B. Tetrahydrofuran (THF) bei - 60 °C vorgelegt und anschließend langsam mit einem Überschuss einer Dicyan-THF-Lösung versetzt. Dabei wird mit einem Überschuss an Dicyan von 1.5 Equivalenten (1.5 eq), bezogen auf die Stoffmenge des vorgelegten Azids gearbeitet. Die Aufarbeitung erfolgt in allen Fällen durch Waschen mit Diethylether und Trocknen im Feinvakuum.

Die Azide können zum Beispiel entsprechend Schritt 1 in Figur 3 durch die Deprotonierung von Stickstoffwasserstoffsäure HN₃ durch verschiedene basische Vorläufer: 1) Bronstedbasen, 2) reaktive Ylide, 3) ionische Flüssigkeiten mit basischen Anionen dargestellt werden. HN₃ kann zum Beispiel *in situ* durch die Reaktion von Trimethylsilylazid (TMS-N₃) mit Methanol (MeOH) in Diethylether generiert werden.

Die Basen werden bei - 60 °C langsam zur Reaktionsmischung zugegeben. Nach langsamem Erwärmen wird für 12 h bei Raumtemperatur gerührt. Die Aufarbeitung erfolgt durch Abfiltrieren des ausgefallenen Azids und Waschen mit Diethylether, wonach die Produkte in quantitativen Ausbeuten erhalten werden.

So können beispielsweise ionische Flüssigkeiten mit Anion A1 und Tetramethylguanidiniumkation, Triethylammoniumkation, Ethylmethylimidazoliumkation, Tributylmethylphosphoniumkation, Butylmethylimidazoliumkation oder Butylmethylpyrrolidiniumkation generiert werden.

Es kann weiterhin eine Methode eingesetzt werden, bei der das Azid ohne vorherige Aufarbeitung direkt zum Tetrazolid umgesetzt wird. Dabei erfolgt nach Lösen des ausgefallenen Azids mit wenig Acetonitril die Zugabe der Dicyan-Lösung ohne vorherige Isolierung des Azids. Die Produkte können durch anschließendes Entfernen aller flüchtigen Bestandteile rein erhalten werden.

Eine weitere Möglichkeit, die Reaktionsvorschrift weiter zu vereinfachen, stellt die Umsetzung in Anwesenheit von Dicyan dar. Dieses reagiert bei Raumtemperatur nicht mit TMS-N₃ oder Methanol.

Entsprechend können Salze mit Metallkationen (Li⁺, Na⁺, Mg²⁺, etc.) und dem Anion A1 durch Reaktion der Metallazide mit Dicyan dargestellt werden.

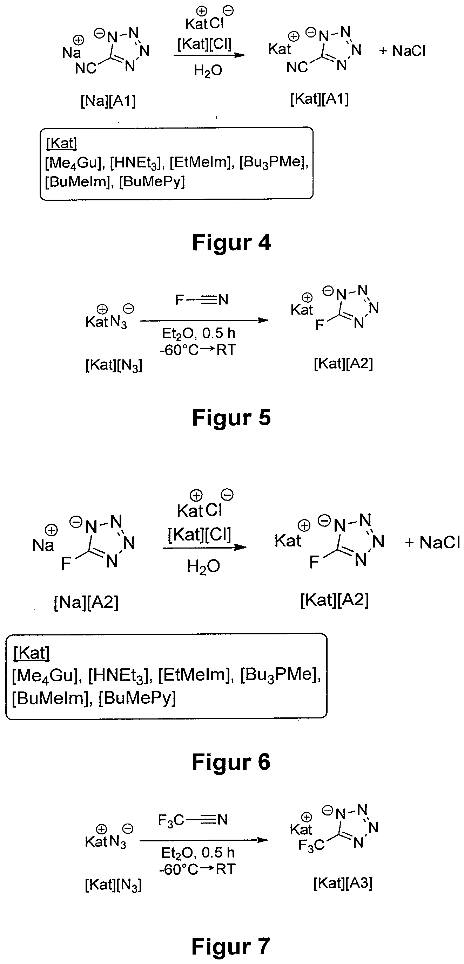

Ein alternativer Syntheseweg ist die Salzaustauschreaktion in Figur 4. Das Alkalimetallsalz von Anion A1 wird in wässriger Lösung vorgelegt und anschließend mit dem Halogenid (zum Beispiel Chloridsalz) des jeweiligen Kations versetzt. Dabei bildet sich die gewünschte [Kat][A1]-ionische Flüssigkeit, die durch mehrmaliges Behandeln mit z. B. CH₂Cl₂ aus der wässrigen Lösung extrahiert werden kann.

### b. Syntheseweg für Anion A2

Gemäß Figur 5 können ionische Flüssigkeiten mit dem Fluortetrazolidanion A2 in einer Cycloadditionsreaktion zwischen den jeweiligen Aziden mit Fluorcyan F-CN generiert werden. Das Azid wird dabei in Lösung in THF bei - 60 °C vorgelegt (Darstellung gemäß Schritt 2 in Figur 3) und anschließend langsam mit einem Überschuss einer Fluorcyan -THF-Lösung versetzt (1.5 eq). Die Aufarbeitung erfolgt durch Waschen mit Diethylether und Trocknen im Feinvakuum. Die Azide können z.B. gemäß Schritt 1 in Figur 3 generiert werden.

Alternativ können Verbindungen des Typs [Kat][A2] durch die Salzaustauschreaktion (Figur 6) aus dem jeweiligen Alkalifluortetrazolid und dem Halogenid des Kations erhalten werden.

### c. Syntheseweg für Anion A3

Gemäß Figur 7 können ionische Flüssigkeiten mit dem Trifluormethyltetrazolidanion A3 in einer Cycloadditionsreaktion zwischen den jeweiligen Aziden mit Trifluormethylcyan CF₃-CN generiert werden. Das Azid wird dabei in Lösung in THF bei - 60 °C vorgelegt (Darstellung gemäß Schritt 2 in Figur 3) und anschließend langsam mit einem Überschuss einer Trifluormethylcyan -THF-Lösung versetzt (1.5 eq). Die Aufarbeitung erfolgt durch Waschen mit Diethylether und Trocknen im Feinvakuum. Die Azide können z.B. gemäß Schritt 1 in Figur 3 generiert werden.

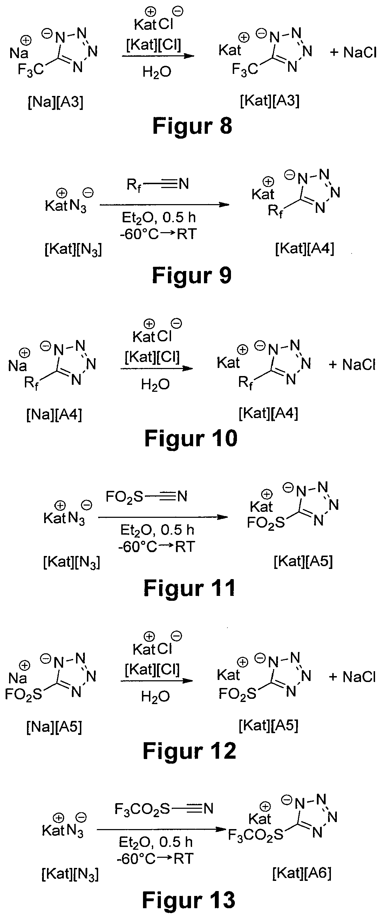

Alternativ können Verbindungen des Typs [Kat][A3] durch die Salzaustauschreaktion (Figur 8) aus dem jeweiligen Alkalitrifluormethyltetrazolid und dem Halogenid des Kations erhalten werden.

Die Halogenidsalze oder auch andere Metallsalze mit Anion A3 können durch die [2+3]-Cycloadditionsreaktion (Figur 3, Schritt 2) aus den Metallaziden mit Trifluormethylcyan dargestellt werden.

### d. Syntheseweg für Anion A4

Gemäß Figur 9 können ionische Flüssigkeiten mit einem Tetrazolidanion A4, das in 5-Position mit einer fluorierten Alkylgruppe substituiert ist, in einer Cycloadditionsreaktion zwischen den jeweiligen Aziden mit Perfluoralkylcyan R_{f}-CN generiert werden. Das Azid wird dabei in Lösung in THF bei - 60 °C vorgelegt (Darstellung gemäß Schritt 2 in Figur 3) und anschließend langsam mit einem Überschuss einer Perfluoralkylcyan R_{f}-CN -THF-Lösung versetzt (1.5 eq). Die Aufarbeitung erfolgt durch Waschen mit Diethylether und Trocknen im Feinvakuum. Die Azide können z.B. gemäß Schritt 1 in Figur 3 generiert werden.

Alternativ können Verbindungen des Typs [Kat][A4] durch die Salzaustauschreaktion (Figur 10) aus dem jeweiligen Alkaliperfluoralkyltetrazolid und dem Halogenid des Kations erhalten werden.

Die Halogenidsalze oder auch andere Metallsalze mit Anion A4 können durch die [2+3]-Cycloadditionsreaktion (Figur 3, Schritt 2) aus den Metallaziden mit Perfluoralkylcyan dargestellt werden.

### e. Syntheseweg für Anion A5

Gemäß Figur 11 können ionische Flüssigkeiten mit einem Tetrazolidanion A5, das in 5-Position mit einer Fluorsulfonylgruppe substituiert ist, in einer Cycloadditionsreaktion zwischen den jeweiligen Aziden mit Fluorsulfonylcyan FSO₂-CN generiert werden. Das Azid wird dabei in Lösung in THF bei - 60 °C vorgelegt (Darstellung gemäß Schritt 2 in Figur 3) und anschließend langsam mit einem Überschuss einer Fluorsulfonylcyan FSO₂-CN ,-THF-Lösung versetzt (1.5 eq). Die Aufarbeitung erfolgt durch Waschen mit Diethylether und Trocknen im Feinvakuum. Die Azide können z.B. gemäß Schritt 1 in Figur 3 generiert werden.

Alternativ können Verbindungen des Typs [Kat][A5] durch die Salzaustauschreaktion (Figur 12) aus dem jeweiligen Alkalifluorsulfonyltetrazolid und dem Halogenid des Kations erhalten werden.

Die Halogenidsalze oder auch andere Metallsalze mit Anion A5 können durch die [2+3]-Cycloadditionsreaktion (Figur 3, Schritt 2) aus den Metallaziden mit Fluorsulfonylcyan dargestellt werden.

### f. Syntheseweg für Anion A6

Gemäß Figur 13 können ionische Flüssigkeiten mit einem Tetrazolidanion A6, das in 5-Position mit einer Trifluormethylsulfonylgruppe substituiert ist, in einer Cycloadditionsreaktion zwischen den jeweiligen Aziden mit Trifluormethylsulfonylcyan CF₃SO₂-CN generiert werden. Das Azid wird dabei in Lösung in THF bei - 60 °C vorgelegt (Darstellung gemäß Schritt 2 in Figur 3) und anschließend langsam mit einem Überschuss einer Trifluormethylsulfonylcyan CF₃SO₂-CN -THF-Lösung versetzt (1.5 eq). Die Aufarbeitung erfolgt durch Waschen mit Diethylether und Trocknen im Feinvakuum. Die Azide können z. B. gemäß Schritt 1 in Figur 3 generiert werden.

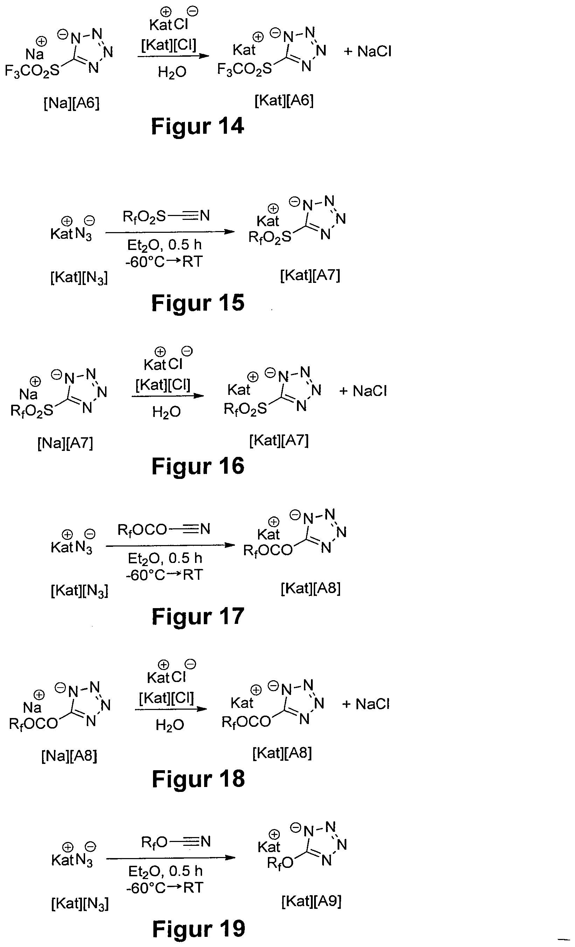

Alternativ können Verbindungen des Typs [Kat][A6] durch die Salzaustauschreaktion (Figur 14) aus dem jeweiligen Alkalitrifluormethylsulfonyltetrazolid und dem Halogenid des Kations erhalten werden.

Die Halogenidsalze oder auch andere Metallsalze mit Anion A6 können durch die [2+3]-Cycloadditionsreaktion (Figur 3, Schritt 2) aus den Metallaziden mit Trifluormethylsulfonylcyan dargestellt werden.

### g. Syntheseweg für Anion A7

Gemäß Figur 15 können ionische Flüssigkeiten mit einem Tetrazolidanion A7, das in 5-Position mit einer Sulfonylgruppe mit höheren perfluorierten Akylrest (-SO₂R_{f}) substituiert ist, in einer Cycloadditionsreaktion zwischen den jeweiligen Aziden mit Perfluoralkylsulfonylcyan R_{f}SO₂-CN generiert werden. Das Azid wird dabei in Lösung in THF bei - 60 °C vorgelegt (Darstellung gemäß Schritt 2 in Figur 3) und anschließend langsam mit einem Überschuss einer Perfluoralkylsulfonylcyan R_{f}SO₂-CN -THF-Lösung versetzt (1.5 eq). Die Aufarbeitung erfolgt durch Waschen mit Diethylether und Trocknen im Feinvakuum. Die Azide können z.B. gemäß Schritt 1 in Figur 3 generiert werden.

Alternativ können Verbindungen des Typs [Kat][A7] durch die Salzaustauschreaktion (Figur 16) aus dem jeweiligen Perfluoralkylsulfonyltetrazolid und dem Halogenid des Kations erhalten werden.

Die Halogenidsalze oder auch andere Metallsalze mit Anion A7 können durch die [2+3]-Cycloadditionsreaktion (Figur 3-Schritt 2) aus den Metallaziden mit Perfluoralkylsulfonylcyan dargestellt werden.

### h. Syntheseweg für Anion A8

Gemäß Figur 17 können ionische Flüssigkeiten mit einem Tetrazolidanion A8, das in 5-Position mit einer Perfluoralkylcarboxylgruppe mit fluorierten Akylrest (-OCOR_{f}) substituiert ist, in einer Cycloadditionsreaktion zwischen den jeweiligen Aziden mit Perfluoralkylcarboxylcyan R_{f}OCO-CN generiert werden. Das Azid wird dabei in Lösung in THF bei - 60 °C vorgelegt (Darstellung gemäß Schritt 2 in Figur 3) und anschließend langsam mit einem Überschuss einer Perfluoralkylcarboxylcyan R_{f}OCO-CN -THF-Lösung versetzt (1.5 eq). Die Aufarbeitung erfolgt durch Waschen mit Diethylether und Trocknen im Feinvakuum. Die Azide können z. B. gemäß Schritt 1 in Figur 3 generiert werden.

Alternativ können Verbindungen des Typs [Kat][A8] durch die Salzaustauschreaktion (Figur 18) aus dem jeweiligen Fluoralkylcarboxyltetrazolid und dem Halogenid des Kations erhalten werden.

Die Halogenidsalze oder auch andere Metallsalze mit Anion A8 können durch die [2+3]-Cycloadditionsreaktion (Figur 3, Schritt 2) aus den Metallaziden mit Perfluoralkylcarboxylcyan dargestellt werden.

### i. Syntheseweg für Anion A9

Gemäß Figur 19 können ionische Flüssigkeiten mit einem Tetrazolidanion A9, das in 5-Position mit einer Üerfluoralkoxygruppe (-OR_{f}) substituiert ist, in einer Cycloadditionsreaktion zwischen den jeweiligen Aziden mit Perfluoralkoxycyan R_{f}O-CN generiert werden. Das Azid wird dabei in Lösung in THF bei - 60 °C vorgelegt (Darstellung gemäß Schritt 2 in Figur 3) und anschließend langsam mit einem Überschuss einer Perfluoralkoxycyan R_{f}O-CN -THF-Lösung versetzt (1.5 eq). Die Aufarbeitung erfolgt durch Waschen mit Diethylether und Trocknen im Feinvakuum. Die Azide können z. B. gemäß Schritt 1 in Figur 3 generiert werden.

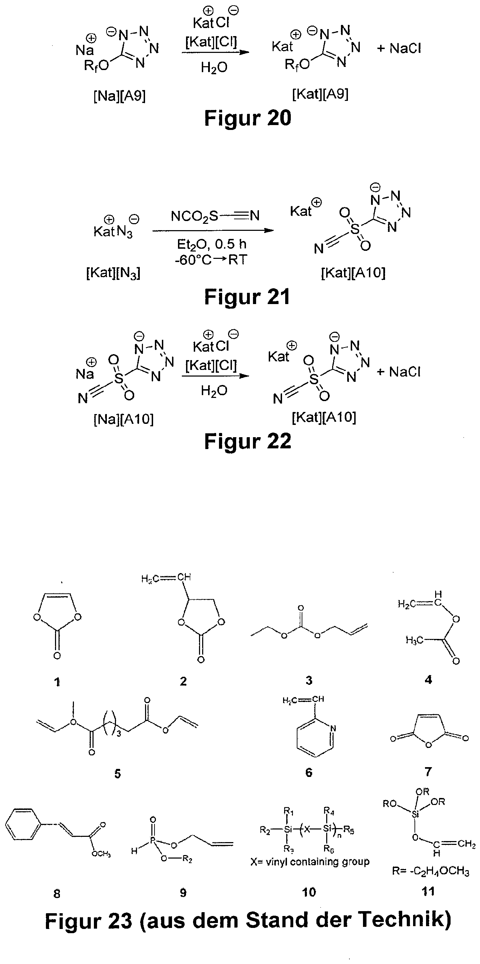

Alternativ können Verbindungen des Typs [Kat][A9] durch die Salzaustauschreaktion (Figur 20) aus dem jeweiligen Perfluoralkoxytetrazolid und dem Halogenid des Kations erhalten werden.

Die Halogenidsalze oder auch andere Metallsalze mit Anion A9 können durch die [2+3]-Cycloadditionsreaktion (Figur 3, Schritt 2) aus den Metallaziden mit Perfluoralkoxycyan dargestellt werden.

### j. Syntheseweg für Anion A10

Gemäß Figur 21 können ionische Flüssigkeiten mit dem Cyanosulfonyltetrazolidanion A10 in einer Cycloadditionsreaktion zwischen den jeweiligen Aziden mit Sulfurylcyanid CN-SO₂-CN generiert werden. Das Azid wird dabei in Lösung in THF bei - 60 °C vorgelegt (Darstellung gemäß Schritt 2 in Figur 3) und anschließend langsam mit einem Überschuss einer Sulfurylcyanid-THF-Lösung versetzt (1.5 eq). Die Aufarbeitung erfolgt durch Waschen mit Diethylether und Trocknen im Feinvakuum. Die Azide können z.B. gemäß Schritt 1 in Figur 3 generiert werden.

Alternativ können Verbindungen des Typs [Kat][A10] durch die Salzaustauschreaktion (Figur 22) aus dem jeweiligen Alkalicyanosulfonyltetrazolid und dem Halogenid des Kations erhalten werden.

Die Halogenidsalze oder auch andere Metallsalze mit Anion A10 können durch die [2+3]-Cycloadditionsreaktion (Figur 3, Schritt 2) aus den Metallaziden mit Sulfurylcyanid dargestellt werden.

### k. Syntheseweg für neue anorganische Salze

Die Darstellung von Salzen der erfindungsgemäßen Anionen mit Metallkationen oder Protonen ist in den Synthesewegen der jeweiligen Anionenverbindungen beschrieben.

### 3. Elektrolytsysteme umfassend erfindungsgemäße ionische Flüssigkeiten oder Salze

Die im Rahmen der Erfindung beschriebenen erfindungsgemäßen Elektrolytsysteme für elektrochemische Bauteile können wie folgt unterteilt werden. Elektrolytsysteme, umfassend
- ausschließlich eine oder mehrere erfindungsgemäße ionische Flüssigkeiten,
- ausschließlich ein oder mehrere erfindungsgemäße Salze,
- Mischungen aus wenigstens einer erfindungsgemäßen ionischen Flüssigkeit und wenigstens einem erfindungsgemäßen Salz,
- Mischungen aus konventionellen Lösungsmitteln und wenigstens einem erfindungsgemäßen Salz,
- Mischungen aus wenigstens einer erfindungsgemäßen ionischen Flüssigkeit, konventionellen Lösungsmitteln und wenigstens einem erfindungsgemäßen Salz,
- einer erfindungsgemäßen Ionischen Flüssigkeit und einem konventionellen Lösungsmittel.
- Polymergel mit wenigstens einer erfindungsgemäßen ionischen Flüssigkeit, Polymergel mit wenigstens einem erfindungsgemäßen Salz,
- Polymergel mit wenigstens einer erfindungsgemäßen ionischen Flüssigkeit und wenigstens einem erfindungsgemäßen Salz,
- Polymergel mit konventionellen Lösungsmitteln und wenigstens einem erfindungsgemäßen Salz,
- Polymergel mit wenigstens einer erfindungsgemäßen ionischen Flüssigkeit, konventionellen Lösungsmitteln und wenigstens einem erfindungsgemäßen Salz.
Die im Folgenden beschriebenen Systeme sind ausgewählte Beispiele, die Tetrazolidanionen aus der Gruppe A1 bis A10, zusammen mit Kationen K1 bis K10 zur Ausbildung von ionischen Flüssigkeiten und/oder Kationen S1 bis S8 zur Ausbildung von Salzen enthalten können. Weiterhin können die Konzentrationen der Salze und Mischungsverhältnisse der verschiedenen Spezies in weiten Bereich eingestellt werden.

### a. Reine ionische Flüssigkeiten

Die im Rahmen dieser Erfindung bereitgestellten reinen ionischen Flüssigkeiten basieren jeweils auf einem Anionen A2 bis A10 in Kombination mit einem der verschiedenen Kationen K1 bis K8 und können gemäß dem angegebenen Syntheseweg dargestellt werden. Es können insbesondere auch Mischungen von herkömmlichen ionischen Flüssigkeiten zusammen mit wenigstens einer erfindungsgemäßen ionischen Flüssigkeit verwendet werden.

Zum Beispiel kann eine Mischung aus herkömmlichen [BuMePy][TFSI] und das nicht erfindungsgemäße [BuMePy][A1] im Verhältnis 1:1 eingesetzt werden. Solche Elektrolyte könnten vorteilhaft in erster Linie in Kondensatoren zum Einsatz kommen.

### b. Mischungen aus ionischen Flüssigkeiten und Salzen

Für eine Mischung aus wenigstens einer erfindungsgemäßen ionischen Flüssigkeit und einem Salz, werden wenigstens eine ionische Flüssigkeit, die aus den Anionen A2 bis A10 und verschiedenen Kationen K1 bis K8 besteht, mit konventionellen Salzen (LiPF₆, LiBF₄, LiBOB, ...) und/oder erfindungsgemäßen Salzen gemischt.
Auch hier können zudem Mischungen von erfindungsgemäßen Tetrazolid-basierten ionischen Flüssigkeiten mit herkömmlichen ionischen Flüssigkeiten eingesetzt werden.
Die Salzkonzentration bezogen auf das Gewicht der ionischen Flüssigkeit(en) beträgt vorteilhaft zwischen 0,1 und 1 mol/kg. Höhere Salzkonzentrationen in Bereich von 1,1 mol/kg bis 3 mol/kg können zwar eingesetzt werden, führen in der Regel aber zu höheren Viskositäten. Die höhere Viskosität beeinflusst die Gesamtionenleitfähigkeit negativ. Allerdings führen höhere Konzentrationen zu höheren Ionentransportzahlen, die sich vorteilhaft auf das elektrochemische Bauteil auswirken.
Durch eine Unterdrückung des Schmelzens werden zudem erweiterte Flüssigkeitsbereiche erhalten. Das Phasenverhalten (Schmelzen, Kristallisieren, Glasübergang) der reinen ionischen Flüssigkeiten kann sich bei der Zugabe eines Salzes signifikant verändern. Insofern können die Flüssigkeitsbereiche der reinen ionischen Flüssigkeiten gegebenenfalls durch die Zugabe von erfindungsgemäßen und konventionellen Salzen vorteilhaft erweitert werden.
Die erfindungsgemäßen Elektrolytsysteme können insbesondere in Batterien, z. B. Lithiumbatterien (LiBs), Farbstoffsolarzellen (engl.: dye-sensitized solar cell, DSSCs), Duallonbatterien, Redoxflusszellen oder auch in Brennstoffzellen eingesetzt werden. Dies gilt für alle Elektrolyte, in denen wenigstens ein Leitsalz enthalten ist, da ohne dieses eine Lithiumionen oder beispielsweise Iodidionenleitfähigkeit nicht gegeben ist.

### c. Mischungen aus konventionellen Lösungsmitteln und erfindungsgemäßen Salzen

Erfindungsgemäß werden konventionelle Lösungsmittel mit erfindungsgemäßen Salzen aus Anionen (A2 bis A10) in Kombination mit Kationen (S1 bis S8) gemischt. Die Salzkonzentration bezogen auf das Gewicht des Lösungsmittels beträgt vorteilhaft zwischen 0,1 und 1 mol/kg. Höhere Salzkonzentrationen in Bereich von 1,1 mol/kg bis 3 mol/kg können zwar eingesetzt werden, führen in der Regel aber zu höheren Viskositäten. Die höhere Viskosität beeinflusst die Gesamtionenleitfähigkeit negativ. Allerdings führen höhere Konzentrationen zu höheren Ionentransportzahlen, die sich vorteilhaft auf das elektrochemische Bauteil auswirken.
Auch diese Elektrolytsysteme können insbesondere in Batterien, z. B. Lithiumbatterien (LiBs), Farbstoffsolarzellen (engl.: dye-sensitized solar cell, DSSCs), Dual-Ionbatterien, Redoxflusszellen oder auch in Brennstoffzellen eingesetzt werden.

### d. Mischungen aus ionischen Flüssigkeiten, konventionellen Lösungsmitteln und Salzen

Durch die Mischung von konventionellen ionischen Flüssigkeiten oder erfindungsgemäßen ionischen Flüssigkeiten, die aus den Anionen A2 bis A10 und den verschiedenen Kationen K1 bis K8 bestehen, mit konventionellen Lösungsmitteln (Wasser oder organische Lösungsmittel) und ferner mit konventionellen Salzen (LiPF₆, LiBF₄, LiBOB,...) oder erfindungsgemäßen Salzen werden Elektrolytsysteme vor allem für Batterien, z. B. Lithiumbatterien (LiBs), Farbstoffsolarzellen (englisch: dye-sensitized solar cell, DSSCs) Dual-Ionbatterien, Redoxflusszellen oder auch in Brennstoffzellen eingesetzt werden.

Die Salzkonzentration bezogen auf das Gewicht des Lösungsmittels und das der ILs beträgt vorteilhaft zwischen 0,1 und 1 mol/kg. Höhere Salzkonzentrationen in Bereich von 1,1 mol/kg bis 3 mol/kg können zwar eingesetzt werden, führen in der Regel aber zu höheren Viskositäten. Die höhere Viskosität beeinflusst die Gesamtionenleitfähigkeit negativ. Allerdings führen höhere Konzentrationen zu höheren Ionentransportzahlen, die sich vorteilhaft auf das elektrochemische Bauteil auswirken.
Das Mischungsverhältnis von ionischer Flüssigkeit zu Lösungsmittel beträgt in der Regel 1:1, kann jedoch über einen weiten Bereich bis hin zu einer reinen Flüssigkeit oder einem reinen Lösungsmittel variiert werden.

Die erfindungsgemäßen Tetrazolid-basierten ionischen Flüssigkeiten und Salze können einzeln oder auch zusammen mit weiteren Additiven in geringen Konzentrationen von 0,005 mol/kg bis 0,1 mol/kg zugesetzt werden. Der geringe Anteil erklärt sich dadurch, dass Additive keinen Anteil am eigentlichen Ionentransport haben, sondern für den Elektrolyten in der Regel nur einen sekundären Einfluss auf das Bauteil haben. Beispielsweise formen sie schützende Oberflächenschichten und führen somit zu erhöhter Sicherheit oder geringerer Alterung.

Additive sind Zusätze in Elektrolyten, die immer in geringen Konzentrationen zwischen 0,005 bis 0,1 mol/kg bezogen auf das Gewicht des Elektrolyten zugesetzt werden. Während sie am normalen Leitungsprozess des Elektrolyten in der Regel keinen Anteil haben, haben sie oft wesentliche Effekte auf Lebensdauer (durch die Bildung von Passivierungsschichten) oder Sicherheitseigenschaften (Brennbarkeit, Überladungsschutz,...).Weiterhin können sie auch Auswirkungen auf die Leitfähigkeit haben.

Konventionelle Additive, die im Rahmen der Erfindung den erfindungsgemäßen Elektrolytsystemen zugesetzt werden können, sind beispielsweise:
- anorganische Partikel: Al₂O₃, SiO₂, AIN, TiN, (oft nanodimensioniert).
- organische Moleküle: Vinylcarbonat, etc., die zur Bildung von stabilen Polymerschichten führen (siehe auch Figur 23).

### e. Polymergel mit erfindungsgemäßer ionischer Flüssigkeit und/oder konventionellem Lösungsmitteln und konventionellem oder erfindungsgemäßem Salz.

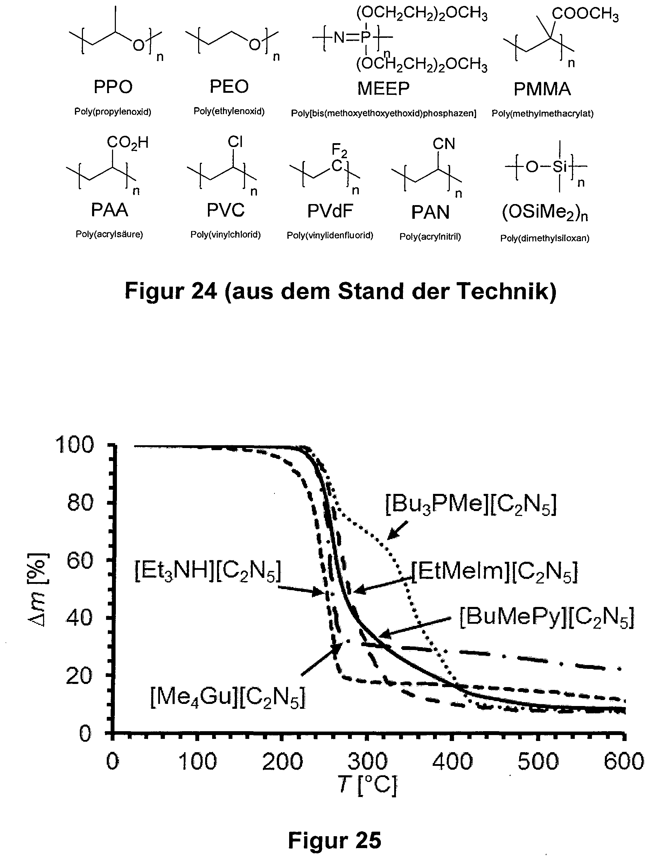

Im Rahmen der Erfindung wurde herausgefunden, dass diverse bekannte Polymere (siehe Figur 24) als Matrix für die erfindungsgemäßen Elektrolyte bzw. Elektrolytsysteme geeignet sind.

### Spezieller Beschreibungsteil

Nachfolgend wird die Erfindung und dabei insbesondere die neuartigen Elektrolytsysteme anhand von Untersuchungen zu Eigenschaften beispielhafter Verbindungen in Form von Tabellen und Figuren näher erläutert, ohne dass dadurch einen Einschränkung im Schutzbereich erfolgen soll.
Dabei werden die thermischen, elektrochemischen und physikalischen Eigenschaften verschiedener reiner ionischer Flüssigkeiten mit Anion A1 (nicht erfindungsgemäß) sowie von Mischungen aus ionischen Flüssigkeiten mit dem Anion A1 (nicht erfindungsgemäß) und dem konventionellen Lithiumbisoxalatoboratsalz (LiBOB) charakterisiert.

### a. Chemische Struktur und Benennung von beispielhaft ausgewählten erfindungsgemäßen (A2 bis A10) und nicht erfindungsgemäßen (A1) Ionischen Flüssigkeiten und Salzen

### Anion A1 (nicht erfindungsgemäß - dient nur als Referenz)

### Konkrete Beispiele: Ethylmethylimidazolium-5-cyanotetrazolid, Butylmethylpyrrolidinium-5-cyanotetrazolid

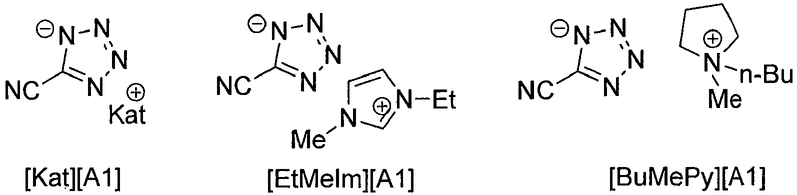

### Anion A2

### Konkrete Beispiele: Ethylmethylimidazolium-5-fluortetrazolid, Butylmethylpyrrolidinium-5-fluortetrazolid

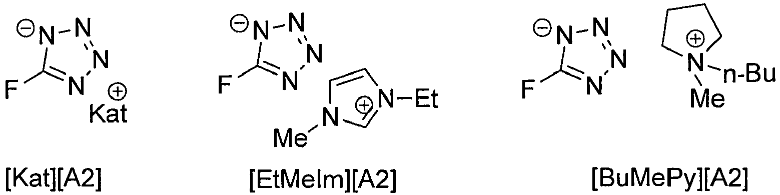

### Anion A3

### Konkrete Beispiele: Ethylmethylimidazolium-5-trifluormethyltetrazolid, Butylmethylpyrrolidinium-5-trifluormethyltetrazolid

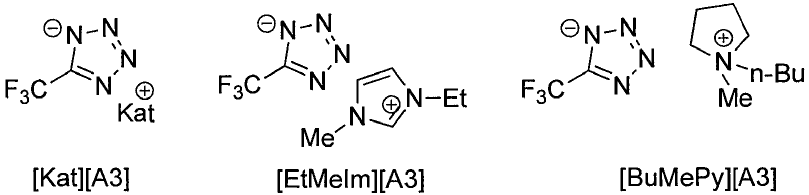

### Anion A4

### Konkrete Beispiele: Ethylmethylimidazolium-5-pentafluorethyltetrazolid, Butylmethylpyrrolidinium-5-pentafluorethyltetrazolid

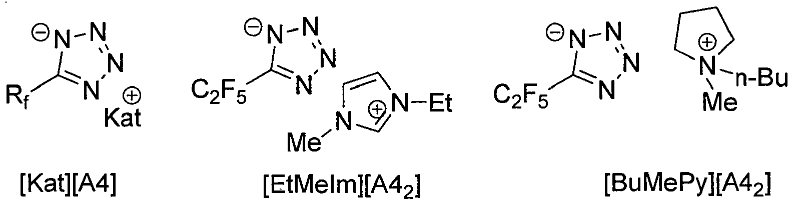

### Anion A5

### Konkrete Beispiele: Ethylmethylimidazolium-5-fluorsulfonyltetrazolid, Butylmethylpyrrolidinium-5-fluorsulfonyltetrazolid

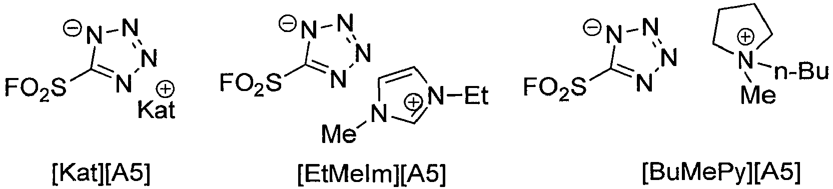

### Anion A6

### Konkrete Beispiele: Ethylmethylimidazolium-5-trifluormethylsulfonyltetrazolid, Butylmethylpyrrolidinium-5-trifluormethylsulfonyltetrazolid

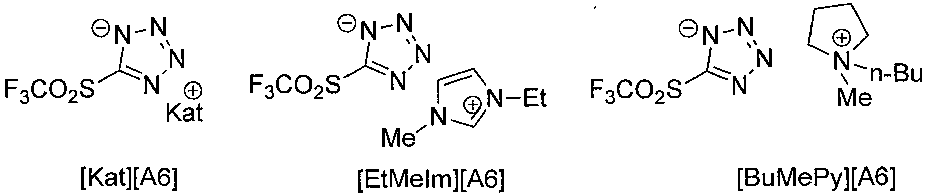

### Anion A7

### Konkrete Beispiele: Ethylmethylimidazolium-5-pentafluorethylsulfonyltetrazolid, Butylmethylpyrrolidinium-5-pentafluorethylsulfonyltetrazolid

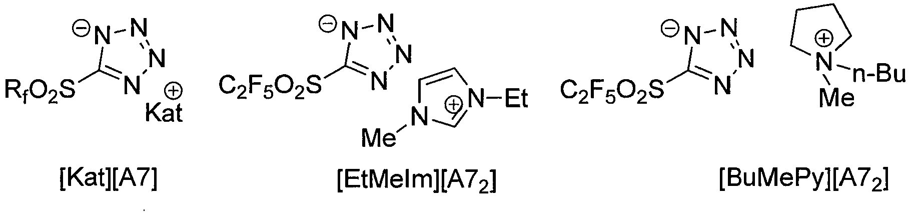

### Anion A8

### Konkrete Beispiele: Ethylmethylimidazolium-5-pentafluorethylcarboxyltetrazolid, Butylmethylpyrrolidinium-5-pentafluorethylcarboxyltetrazolid

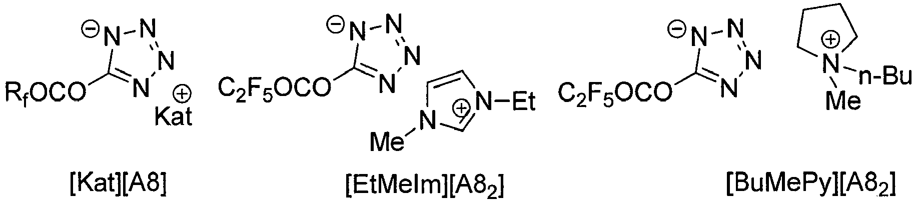

### Anion A9

### Konkrete Beispiele: Ethylmethylimidazolium-5-pentafluorethyoxytetrazolid, Butylmethylpyrrolidinium-5-pentafluorethyoxytetrazolid

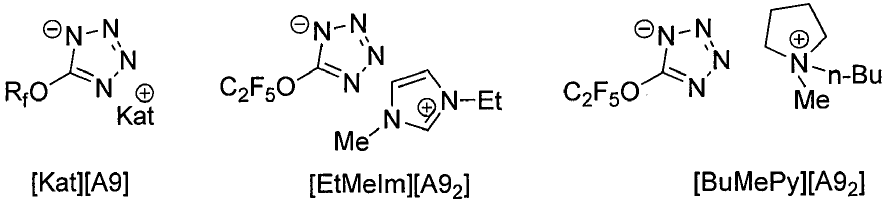

### Anion A10

### Konkrete Beispiele: Ethylmethylimidazolium-5-cyanosulfonyltetrazolid, Butylmethylpyrrolidinium-5-cyanosulfonyltetrazolid

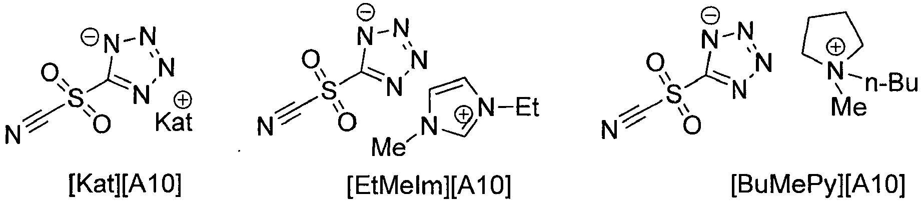

### b. Eigenschaften der erfindungsgemäßen ionischen Flüssigkeiten und Salze

Die nachfolgend erläuterten Eigenschaften der vorgenannten Systeme stehen stellvertretend für alle übrigen erfindungsgemäßen offenbarten ionischen Flüssigkeiten und Salze.

Die Charakterisierung der ionischen Flüssigkeit, die das nicht erfindungsgemäße Anion A1 umfasst, erfolgte wie folgt:
- Infrarotspektroskopie: Es wurde eine starke, für das Tetrazolid-Anion charakteristische Bande der CN-Funktion bei etwa 2240 cm⁻¹ für die symmetrische Valenzschwingung ermittelt.
- Kernspinresonanzspektroskopie (NMR): Das C-Atom der Cyanofunktion weist im ¹³C-NMR eine Verschiebung von etwa 114 ppm auf, während für jenes im Tetrazolid-Ring ein Singulett bei 139 ppm beobachtet wird.
- Chemische Analyse: Die ionische Flüssigkeit umfassend [EtMelm][A1] weist eine Molmasse von 205,22 g/mol auf. Es wurde ein C-Anteil von 46,82 %, ein H-Anteil von 5,40 % und ein N-Anteil von 47,78 % gefunden. Diese Werte stimmen gut mit den theoretischen Werten überein.
- Röntgendiffraktion (XRD): Die Bindungslängen und -winkel des Anions A1 sind in den Tabellen 4 und 5 im Anhang für verschiedene Verbindungen aufgelistet. Die Atombenennung des Anions A1 ist ebenfalls aufgeführt. Die Bindungsverhältnisse variieren kaum mit einem Wechsel des Kations.
- Elektrospray-lonisations-Massenspektrometrie (ESI-MS): Das MS-Signal des Tetrazolidanions A1 in der ionischen Flüssigkeit [EtMelm][A1] in Dichlormethan als Lösungsmittel erscheint bei 94,1 g/mol.

### c. Untersuchungen zur thermischen Stabilität

Die thermische Stabilität von ionischen Flüssigkeiten auf Basis des nicht erfindungsgemäßen Anions A1 = [C₂N_{5]} ist weitestgehend durch Zersetzung des Anions begrenzt und liegt etwa bei T_{Dekomp} = 240 °C. Im Anhang sind die Verläufe der thermogravimetrischen Analysen verschiedener ionischer Flüssigkeiten unter N₂-Atmosphäre aufgezeigt (siehe Figur 25). Die thermische Zersetzung beginnt unmittelbar nach der genannten Dekompositionstemperatur und läuft in der Regel bis zu einem Restmassenanteil von etwa 10 Gew.-% bei 600 °C einstufig ab.
Die Werte sind vergleichbar mit denen von herkömmlichen FSI-basierten ionische Flüssigkeiten (T_{Dekomp}([BuMePy][FSI]) = 180 °C).

Weiterhin wurde ein TGA-Experiment mit [EtMelm][A1] (nicht erfindungsgemäß) unter O₂-Atmosphäre durchgeführt. Die Probe zeigt ein sehr ähnliches Verhalten wie unter N₂-Atmosphäre. Es werden also für alle Verbindungen, auch unter atmosphärischen Bedingungen relativ hohe thermische Belastbarkeiten vorausgesagt. Liegt eine Verbindung mit einem protischen Kation wie etwa Triethylammonium vor, ist die thermische Stabilität signifikant verringert (T_{Dekomp} = 120 °C). Dies ist wahrscheinlich auf ein Säure-Base-Gleichgewicht zwischen der ionischen Flüssigkeit und dem jeweiligen Amin und Tetrazol zurückzuführen.
Weitere Ergebnisse der TGA-Experimente sind im Anhang unter der Tabelle 2 zu finden.
Mischungen von ionischen Flüssigkeiten mit LiBOB weisen vergleichbare thermische Stabilitäten wie die reinen ionische Flüssigkeiten auf.
Somit weisen die erfindungsgemäßen Elektrolytsysteme umfassend wenigstens eine ionische Flüssigkeit mit oder ohne Salz eine deutlich höhere thermische Stabilität als konventionelle Elektrolyte für die Anwendung in elektrochemischen Bauteilen auf. Weiterhin weisen sie einen vernachlässigbaren Dampfdruck und einen Flammpunkt oberhalb von 200 °C auf. Die Sicherheitseigenschaften in Lithiumbatterien (LiBs) sind durch die erfindungsgemäßen Elektrolyte daher gegenüber konventionellen Elektrolyten auf Basis organischer Lösungsmittel optimiert.

### d. Phasenverhalten

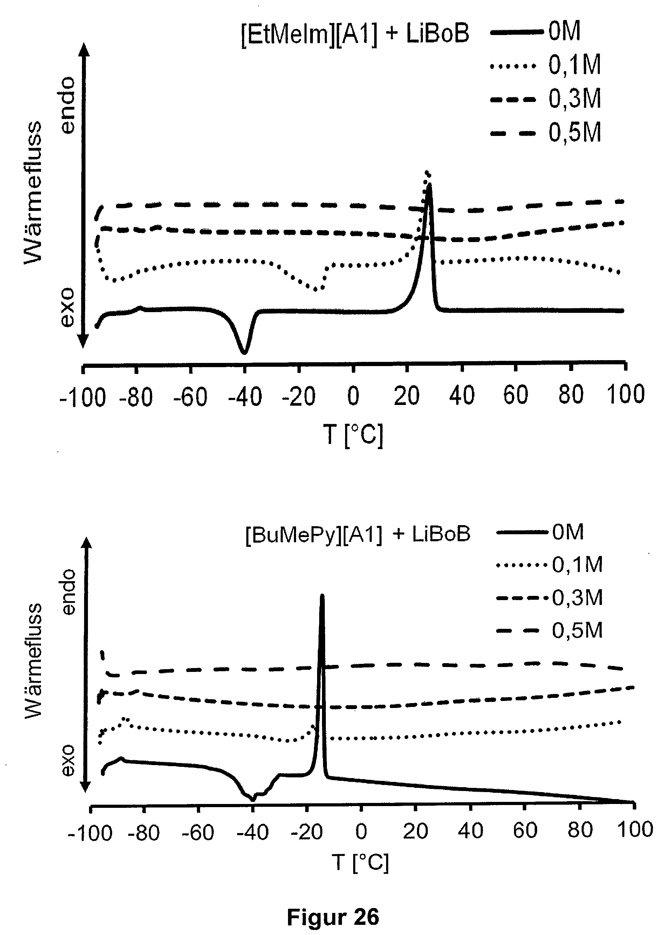

Das Phasenverhalten von [BuMePy][A1] (nicht erfindungsgemäß) in Abhängigkeit von der Konzentration an Lithiumbis(oxalato)borat (LiBOB) als Leitsalz ist in der Figur 26 (unten) im Anhang aufgezeigt. Die reine ionische Flüssigkeit zeigt einen klar definierten Schmelz- und Kristallisationsübergang bei -13 und -30 °C. Der Flüssigkeitsbereich ist im Vergleich zu [EtMelm][A1] deutlich erhöht (T_{Smp} = 31 °C, siehe Figur 26 (oben)). Zusätzlich hat die reine ionische Flüssigkeit einen Glasübergang bei T_{g} = - 87 °C.
Bei Erhöhung der LiBOB-Konzentration wird der Schmelz- und Kristallisationsübergang unterdrückt. So wird für eine Konzentration von 0,1 mol/kg eine im Vergleich zur reinen ionischen Flüssigkeit stark verringerte Schmelzenthalpie charakterisiert, während die Kristallisation vollständig ausbleibt. Für erhöhte Salzkonzentrationen werden durchweg weder ein Kristallisations- noch ein Schmelzübergang beobachtet, so dass der Flüssigkeitsbereich der Elektrolyte mit erfindungsgemäßen ionischen Flüssigkeiten vorteilhaft signifikant erweitert ist. Die Glasübergangstemperatur steigt mit zunehmendem Salzgehalt an.

Der Wert fürT_{g} der[BuMePy][A1] (nicht erfindungsgemäß) umfassenden Elektrolyte reicht von T_{g}(01, mol/kg) = -85 °C für eine LiBOB-Konzentration von 0,1 M, über T_{g}(0,3 mol/kg) = -80 °C für eine LiBOB-Konzentration von 0,3 mol/kg, hin zu T_{g}(0,5 mol/kg) = -78 °C für eine LiBOB-Konzentration von 0,5 mol/kg.
Weitere Ergebnisse der DSC-Versuche sind im Anhang unter der Tabelle 3 zu finden.
Somit weisen Elektrolyte mit wenigstens einer erfindungsgemäßen ionischen Flüssigkeit und mit Salzkonzentrationen oberhalb von 0,1 mol/kg eine deutlich geringere Minimaltemperatur als konventionelle Elektrolyte für die Anwendung in elektrochemischen Bauteilen auf.

### e. Elektrochemische Stabilität

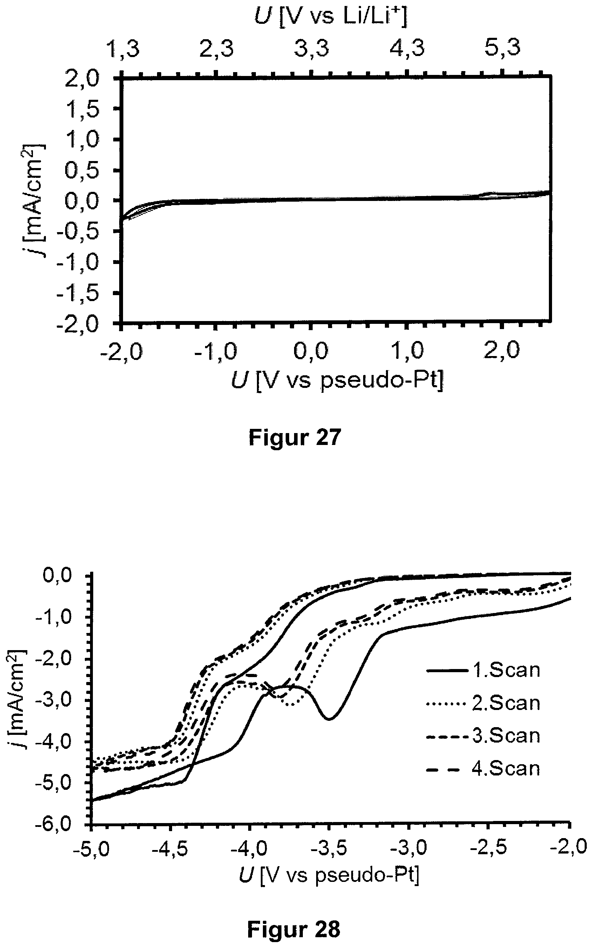

Das Cyclovoltammogramm von [BuMePy][A1] (nicht erfindungsgemäß) an Pt-Elektroden ist beispielhaft in Figur 27 im Anhang skizziert. Es wird ein Reduktionspotential (ϕ_{Red}) von - 2 V gegenüber einer Pt-Bezugselektrode, die in der Probe eintauchte, und ein Oxidationspotential (ϕ_{Ox}) von 2,5 V gegenüber einer Pt-Bezugselektrode, die in der Probe eintauchte, charakterisiert. Die entsprechenden Werte gegenüber Lithium können - nach Kalibrierung mit einem Ferrocen-Ferrocerium-Redoxgleichgewicht - mit ϕ_{Red} = 1,4 V und ϕ_{Ox} = 5,8 V angegeben werden.
Verglichen mit ionischen Flüssigkeiten auf Basis von [BuMePy]-Kationen, wie [BuMe-Py][TFSI] (ϕ_{Red} = - 0,1 V; ϕ_{Ox} = 5,2 V) ^{[4]} oder [BuMePy][FSI] (ϕ_{Red} = 0 V; ϕ_{Ox} = 5,4 V) ^{[5]}, kann die Stabilität gegenüber Oxidation erweitert werden, während die Stabilität gegenüber Reduktion vermindert ist. Die Verringerung der kathodischen Stabilität ist wahrscheinlich auf eine leichtere Reduzierbarkeit des Anions zurückzuführen. Ab - 4,8 V setzt jedoch eine diffusionskontrollierte Abnahme der Stromdichte ein (siehe Figur 28). In aufeinanderfolgenden Zyklen nimmt sie zusätzlich sukzessive ab. Diese Beobachtungen sprechen für die Bildung einer Polymerschicht auf den Metallelektroden. Dies wird auf die Generierung einer schützenden Polymerschicht, z. B. von Carbonitriden [CₙNₘ]ₓ, zurückgeführt. Diese bildet in elektrochemischen Bauteilen (z. B. Lithiumionenbatterien) an der Anode eine stabilisierende feste Elektrolytgrenzfläche aus. Eine ähnliche Schichtbildung wurde für die TFSI- und FSI-basierten ionischen Flüssigkeiten nicht berichtet. Ionische Flüssigkeiten, deren Kationen Cyanofunktionen enthalten, zeigen ein ähnliches Verhalten auf Edelstahlelektroden ^{[6]}.
Somit ist die anodische Stabilität der Tetrazolid-basierten ionischen Flüssigkeiten im Vergleich zu konventionellen Lösungsmitteln und ionische Flüssigkeiten erhöht während die kathodische Stabilität reduziert ist. Durch die Bildung einer Polymerschutzschicht werden die erfindungsgemäßen Verbindungen jedoch vor der Reduktion geschützt, so dass die Stabilität bei hohen anodenseitigen Potentialen in elektrochemischen Bauteilen gegeben ist. Dies wird durch die Möglichkeit der wiederholten Auflösung und Abscheidung von Li⁺ an stark reduzierenden Lithiumelektroden in einem Elektrolyten (nicht erfindungsgemäß) umfassend [BuMePy][A1] und LiBOB-Leitsalz bestätigt. Die erhöhte anodische Stabilität führt dazu, dass die erfindungsgemäßen Elektrolyte beim Einsatz von Hochvoltkathoden in Lithiumionenbatterien mit Potentialen gegen Lithium von 5 V besonders vorteilhafte Eigenschaften aufweisen.

### f. Viskosität

Die Viskosität von [EtMelm][A1] (nicht erfindungsgemäß) bei 20 °C (η(20 °C) = 28,6 mPa·s) liegt deutlich unterhalb jener von [EtMelm][TFSI] (η(20 °C) = 37,3 mPa·s). Die Temperaturabhängigkeit der Viskosität kann durch die Vogel-Fulcher-Tammann-Funktion beschrieben werden. Es zeigt sich, dass die Viskosität der erfindungsgemäßen ionische Flüssigkeiten im Vergleich zu konventionellen ionische Flüssigkeiten in der Regel deutlich erniedrigt ist.

### g. Leitfähigkeit

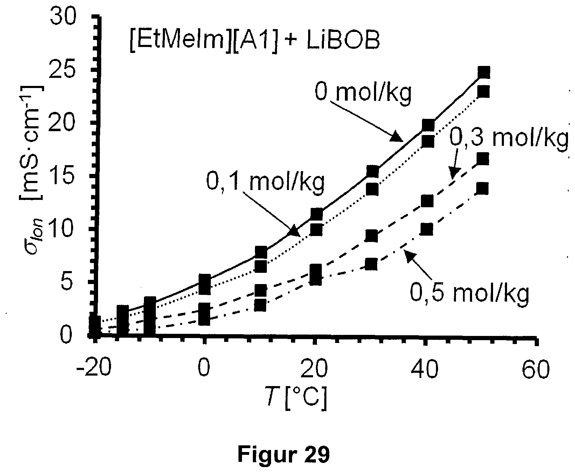

Die temperaturabhängige Gesamtionenleitfähigkeit der nicht erfindungsgemäßen ionischen Flüssigkeit mit [EtMelm][A1] und mit unterschiedlichen Konzentrationen an LiBOB ist in Figur 29 im Anhang skizziert. Die Leitfähigkeit von [EtMelm][A1] oder [A1] liegt mit einem Wert von σ_{Ion},20°c = 11,5 mS/cm bei 20 °C deutlich über der von der konventionellen ionischen Flüssigkeiten, wie beispielsweise [EtMelm][FAP] (σ_{Ion},20°c = 4,9 mS/cm). Die Temperaturabhängigkeit der Leitfähigkeit kann durch die Vogel-Fulcher-Tammann-Funktion beschrieben werden.
Die Erhöhung der Salzkonzentration resultiert in der Verringerung der ionischen Gesamtleitfähigkeit verglichen mit den reinen ionische Flüssigkeiten. Dies kann mit einer zunehmenden Viskosität erklärt werden. [EtMelm][A1] (nicht erfindungsgemäß) weist bei einer LiBOB-Konzentration von 0,3 mol/kg eine Leitfähigkeit von 6,2 mS/cm bei 20 °C auf. Bei - 20 °C werden Leitfähigkeiten von 0,56 mS/cm erhalten. Dieser Wert liegt oberhalb der Leitfähigkeit der meisten bekannten ionischen Flüssigkeiten und kann mit den Werten von Elektrolyten auf Basis konventioneller organischer Lösungsmittel verglichen werden.

Somit weisen die erfindungsgemäßen Elektrolyte höhere Leitfähigkeiten als die meisten konventionellen Elektrolyte mit bekannten ionischen Flüssigkeiten auf.

### h. Anwendung in Lithiumbatterien

Die vorgenannten erfindungsgemäßen beschriebenen Elektrolytsysteme:
- Mischungen aus konventionellen Lösungsmitteln und erfindungsgemäßen Salzen
- Mischungen aus ionischen Flüssigkeiten, konventionellen Lösungsmitteln und Salzen
- Mischungen aus ionischen Flüssigkeiten und Salzen
- Polymergel mit erfindungsgemäßer ionischer Flüssigkeit und/oder konventionellem Lösungsmitteln und konventionellem oder erfindungsgemäßem Salz
können vorteilhaft in Lithiumbatterien mit variablen Elektroden, Stromkollektoren und Passivkomponenten eingesetzt werden.

Zum Beispiel können die Elektrolyte in einer Batterie mit einem Lithiumtitanat-Anodenkomposit und einem Lithiumnickelmanganspinell-Kathodenkomposit eingesetzt werden. Der Anodenkomposit besteht dabei zum Beispiel aus 10 Gew.-% Polyvinylidenfluorid (PvdF als Binder), 10 Gew.-% Leitruß (z. B. Super-P als Leitfähigkeitsadditiv) und 80 Gew.-% des Anodenaktivmaterials (z.B. Li₄Ti₅O₁₂) und wird mittels bekannten Prozeduren auf einer dünnen Kupferfolie zu Elektrodenschichten verarbeitet. Der Kathodenkomposit besteht dabei zum Beispiel aus 10 Gew.-% Polyvinylidenfluorid (PvdF als Binder), 10 Gew.-% Leitruß (z.B. Super-P als Leitfähigkeitsadditiv) und 80 Gew.-% des Kathodenaktivmaterials (z. B. Li-Ni_{0,5}Mn_{1,5}O₄) und wird mittels bekannten Prozeduren auf einer dünnen Aluminiumfolie zu Elektrodenschichten verarbeitet. Zwischen die Elektrodenschichten wird eine dünne Separatorfolie, beispielsweise ein Glasfaserflies, gelegt. Dieser wird mit beispielsweise 200 Mikroliter pro Quadratzentimeter des Elektrolyten benetzt.

### In der Anmeldung zitierte Literatur:

[1] McEwen, A. B.; Ngo, H. L.; LeCompte, K.; Goldman, J. L., Electrochemical Properties of Imidazolium Salt Electrolytes for Electrochemical Capacitor Applications. J. Electrochem. Soc. 1999, 146 (5), 1687-1695.
[2] Profatilova, I. A.; Choi, N.-S.; Roh, S. W.; Kim, S. S., Electrochemical and thermal properties of graphite electrodes with imidazolium- and piperidinium-based ionic liquids. J. Pow. Sourc. 2009, 192 (2), 636-643.
[3] J. Sundermeyer; H. W. Roesky; Noltemeyer, M., Azid Dicyan Tetrazolid Klickreaktion. Z. Naturforsch. B 1990, 45, 77-79.
[4] Jin, J.; Li, H. H.; Wei, J. P.; Bian, X. K.; Zhou, Z.; Yan, J., Li/LiFePO4 batteries with room temperature ionic liquid as electrolyte. Electrochem. Comm. 2009, 11 (7), 1500-1503.
[5] Appetecchi, G. B.; Montanino, M.; Balducci, A.; Lux, S. F.; Winterb, M.; Passerini, S., Lithium insertion in graphite from ternary ionic liquid-lithium salt electrolytes: I. Electrochemical characterization of the electrolytes. J. Pow. Sourc. 2009, 192 (2), 599-605.
[6] Zhao, L.; Yamaki, J.-i.; Egashira, M., Analysis of SEI formed with cyano-containing imidazolium-based ionic liquid electrolyte in lithium secondary batteries. J. Pow. Sourc. 2007, 174 (2), 352-358.
[7] Arp, H. P.; Decken, A.; Passmore, J.; Wood, J. D., Preparation, Characterization, X-ray Crystal Structure, and Energetics of Cesium 5-Cyano-1,2,3,4-Tetrazolate: Cs[NCCNNNN]. Inorg. Chem. 2000, 39 (9), 1840-1848.

### Anhang

**Tabelle 1:**

| R in Anion | Struktur | | Anion |
|---|---|---|---|
| R= -CN | 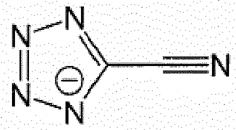 | | A1 (nur zur Referenz) |
| R= -F | 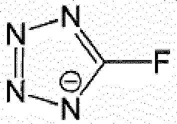 | | A2 |
| R = -CF₃ | 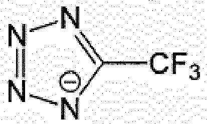 | | A3 |
| R = -R₁ | 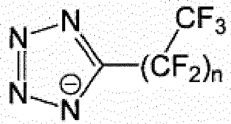 | mit n=1, 2, 3, 4, 5, 6, 7 | A4ₙ |
| R = -SO₂F | 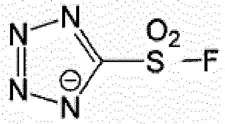 | | A5 |
| R = -SO₂CF₃ | 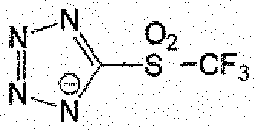 | | A6 |
| R = -SO₂R_{f} | 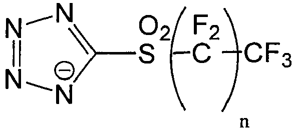 | mit n=1, 2, 3, 4, 5, 6, 7 | A7ₙ |
| R = -OCOR_{f} | 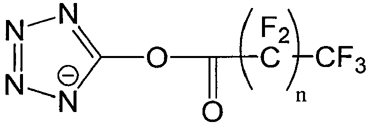 | mit n=1, 2, 3, 4, 5, 6, 7 | A8ₙ |
| R = -OR_{f} | 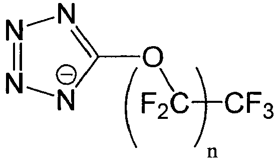 | mit n=1, 2, 3, 4, 5, 6, 7 | A9ₙ |
| R = -SO₂CN | 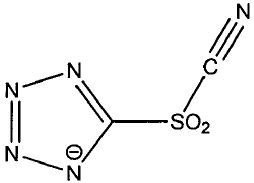 | | A10 |

**Tabelle 2:**

| Ergebnisse der Thermogravimetrischen Analyse (alle untersuchten Substanzen nicht erfindungsgemäß) Temperatur bei der ein Masseverlust von 5 Gew.- % aufgetreten ist (T5%), Restmassenprozent bei 800 °C (Rest-Gew.-%), Zersetzungsbereich vom Beginn bis zum Ende der Dekomposition (ΔT) | | | |
|---|---|---|---|
| | T5% [°C] | Rest-Gew.-% | ΔT[°C] |
| [Bu3PMe][A1] | 235 | 7 | 195 |
| [Ph3PMe][A1] | 237 | 9 | 113 |
| [EtMelm][A1] | 231 | 4 | 105 |
| [BuMelm][A1] | 225 | 9 | 125 |
| [iPr2lm][A1]) | 232 | 3 | 102 |
| [HNEt3][A1]) | 188 | 2 | 118 |
| [Bu4N][A1] | 207 | 5 | 116 |
| [Me4Gu][A1] | 228 | 17 | 84 |
| [BuMe4Gu][A1] | 222 | 9 | 81 |

**Tabelle 3:**

| Ergebnisse der Dynamischen Differenzkalorimetrie (DSC): Schmelzpunkte (T_{Smp}) und - enthalpien (H_{Smp}), Kristallisationspunkte (Tₖᵣᵢₛₜ) und -enthalpien (Hₖᵣᵢₛₜ) (alle untersuchten Substanzen nicht erfindungsgemäß) | | | | |
|---|---|---|---|---|
| | T_{Smp} [°C] | H_{Smp} [J/g] | Tₖᵣᵢₛₜ [°C] | Hₖᵣᵢₛₜ [J/g] |
| [Bu3PMe][A1] | 30 | -58 | 0 | 68*1 |
| [Ph3PMe][A1] | 95 | -54 | 49 | 48 |
| [EtMelm][A1] | 17 | -107 | -51 | 59 |
| [BuMelm][A1] | 12 | -69 | -20 | 77 |
| [iPr2lm][A1] | 30 | -58 | -22 | 52 |

**Tabelle 4:**

| Ausgewählte Bindungslängen in A von [EtMelm][C₂N₅], [HNEt₃][C₂N₅], [Bu₃PMe][C₂N₅] und Cs[C₂N₅] | | | | |
|---|---|---|---|---|
| | [EtMelm][C₂N₅] | [HNEt₃][C₂N₅] | [Bu₃PMe][C₂N₅] | Cs[C₂N₅]^{[7]} |
| N(25)-C(22) | 1.142(2) | 1.148(1) | 1.144(2) | 1.137(1) |
| C(22)-C(21) | 1.432(2) | 1.432(1) | 1.428(2) | 1.430(1) |
| C(21)-N(24) | 1.325(2) | 1.337(1) | 1.321(2) | 1.325(1) |
| N(24)-N(23) | 1.331(2) | 1.338(1) | 1.341(2) | 1.333(1) |
| N(23)-N(22) | 1.323(2) | 1.326(1) | 1.304(2) | 1.303(1 |
| N(22)-N(21) | 1.335(2) | 1.326(1) | 1.342(2) | 1.333(1) |
| N(21)-C(21) | 1.333(2) | 1.337(1) | 1.331(2) | 1.325(1) |

**Tabelle 5:**

| Ausgewählte Bindungswinkel in ° von [EtMelm][C₂N₅], [HNEt₃][C₂N₅], [Bu₃PMe][C₂N₅] und Cs[C₂N₅]. | | | | |
|---|---|---|---|---|
| | [EtMelM][C₂N₅] | [HNEₜ₃][C₂N5] | [Bu₃PMe][C₂N₅] | Cs[C₂N₅]^{[7]} |
| N(25)-C(22)-C(21) | 178.9(2) | 179.2(1) | 178.7(2) | 180.0(0) |
| N(21)-C(21)-C(22) | 122.6(1) | 123.1(1) | 124.1(1) | 123.6(3) |
| N(21)-C(21)-N(24) | 113.9(2) | 113.7(1) | 113.0(1) | 112.7(6) |
| N(24)-N(23)-N(22) | 110.2(1) | 110.6(1) | 109.7(1) | 109.8(3) |
| N(23)-N(22)-N(21) | 109.3(1) | 109.2(1) | 109.8(1) | 109.8(3) |
| N(24)-C(21)-C(22) | 123.4(1) | 123.1(1) | 122.8(1) | 123.6(3) |
| C(21)-N(24)-N(23) | 103.1(1) | 102.7(1) | 103.9(1) | 103.8(4) |
| C(21)-N(21)-N(22) | 103.2(1) | 103.6(1) | 103.5(1) | 103.8(3) |

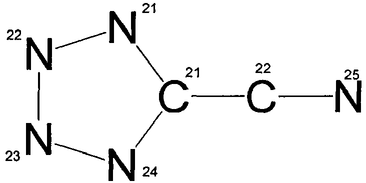

Strukturformel von Anion A1 (nicht erfindunasgemäß) mit Benennung der Atome für Tabelle 4 und Tabelle 5

## Patentansprüche

1. Elektrolytsystem für den Einsatz in einem elektrochemischen Bauteil, umfassend wenigstens ein Tetrazolidanion A mit einem Substituenten R in 5-Position gemäß der folgenden Formel 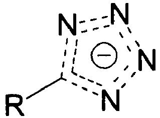 sowie wenigstens ein Kation,
***dadurch gekennzeichnet,***
**dass** der Substituent R aus der Gruppe:
a) R = Fluorgruppe (-F),
b) R = Trifluormethylgruppe (-CF₃),
c) R = Alkylrest (R_{f}), der vollständig oder teilweise fluoriert ist mit C1 bis C8, wobei der Alkylrest linear oder verzweigt sein kann,
d) R = ein durch einen Elektronen-ziehenden Substituenten substituierte Sulfonylgruppe,
e) R = ein durch einen Elektronen-ziehenden Substituenten substituierte Carboxylgruppe,
f) R = ein durch einen Elektronen-ziehenden Substituenten substituierte Ethergruppe,
g) R = Cyanosulfonylgruppe (-SO₂CN)
gewählt ist, und dass das Elektrolytsystem wenigstens ein Kation K_{IL} aus der Gruppe:
a) K_{IL} = Ammoniumkationen mit verschiedenen Resten (NRR¹R²R³),
mit R, R¹, R², R³ = -(CH₂)ₙ-CH₃ und n = 0, 1, 2, 3, 4, 5, 6, 7, wobei die Reste R, R¹, R², R³ unterschiedlich oder auch gleich sein können,
b) K_{IL} = Imidazoliumkationen mit verschiedenen Resten in 1-, 2-, 3-, 4- und 5-Position (RR¹R²R³R⁴lm), mit R, R¹, R², R³, R⁴ = H, -(CH₂)ₙ-CH₃ und n = 0, 1, 2, 3, 4, 5, 6, 7, wobei die Reste R, R¹, R², R³, R⁴ unterschiedlich oder auch gleich sein können,
c) K_{IL} = Pyrrolidiniumkationen mit verschiedenen Resten in 1-Position (RR¹Py),
mit R, R¹ = -(CH₂)ₙ-CH₃ und n = 0, 1, 2, 3, 4, 5, 6, 7, wobei die Reste R, R¹ unterschiedlich oder auch gleich sein können,
d) K_{IL} = Piperidiniumkationen mit verschiedenen Resten in 1-Position (RR¹Pip),
mit R, R¹ = -(CH₂)ₙ-CH₃ und n = 0, 1, 2, 3, 4, 5, 6, 7, wobei die Reste R und R¹ unterschiedlich oder auch gleich sein können,
e) K_{IL} = Pyridiniumkationen mit verschiedenen Resten in 1-Position (RPyri),
mit R = -(CH₂)ₙ-CH₃ und n = 0, 1, 2, 3, 4, 5, 6, 7,
f) K_{IL} = Pyrazoliumkationen mit verschiedenen Resten in 1-, 2-, 3-, 4- und 5-Position (RR¹R^{z}R³R⁴Pyr), mit R, R¹, R², R³, R⁴ = H, -(CH₂)ₙ-CH₃ und n = 0, 1, 2, 3, 4, 5, 6, 7, wobei die Reste R, R¹, R², R³, R⁴ unterschiedlich oder auch gleich sein können,
g) K_{IL} = Phosphoniumkationen mit verschiedenen Resten (PRR¹R²R³), mit R, R¹, R², R³ = -(CH₂)ₙ-CH₃ und n = 0, 1, 2, 3, 4, 5, 6, 7, wobei die Reste R, R¹, R², R³ unterschiedlich oder auch gleich sein können,
h) K_{IL} = Sulfoniumkationen mit verschiedenen Resten (SRR¹R²), mit R, R¹, R² = -(CH₂)ₙ-CH₃ und n = 0, 1, 2, 3, 4, 5, 6, 7, wobei die Reste R, R¹, R², R³unterschiedlich oder auch gleich sein können,
oder wenigstens ein Kation K_{S} aus der Gruppe:
a) K_{S} = H⁺,
b) K_{S} = Li^{+,},
c) K_{S} = Na⁺,
d) Kₛ = K⁺,
e) K_{S} = Mg²,⁺
f) K_{S} = Ca²⁺,
g) K_{S} = Ni²⁺,
h) K_{S} = Pb²⁺,
i) K_{S} = Fₑ^{2+/3+}
j) Kₛ = V^{2+/3+/4+/5+}
umfasst.

2. Elektrolytsystem nach Anspruch 1 mit einem in 5-Position substituierten Tetrazolidanion A mit einem Substituenten R, wobei R ausgewählt ist aus der Gruppe:
R = Fluorsulfonylgruppe (-SO₂F),
R = Trifluormethylsulfonylgruppe (-SO₂CF₃) oder
R = Sulfonylgruppe mit einem höher fluorierten Akylrest (-SO₂R_{f}) mit R_{f} = -(CF₂)ₙCF₃ und
mit n = 1, 2, 3, 4, 5, 6, oder 7.

3. Elektrolytsystem nach Anspruch 1 mit einem in 5-Position substituierten Tetrazolidanion A mit einem Substituenten R = Carboxylgruppe (-OCOR_{f}) mit R_{f} = (CF₂)ₙCF₃ und mit n = 1, 2, 3, 4, 5, 6, oder 7.

4. Elektrolytsystem nach Anspruch 1 mit einem in 5-Position substituierten Tetrazolidanion A mit einem Substituenten R = Alkoxygruppe (-OR_{f}) mit R_{f} = (CF₂)ₙCF₃ und mit n = 1, 2, 3, 4, 5, 6, oder 7.

5. Verfahren zur Herstellung eines Elektrolytsystems gemäß Anspruch 1,
mit einer [2+3] Cycloadditionsreaktion in organischen Lösungsmitteln zwischen einem ionischen Azid, welches ein erfindungsgemäßes Kation K_{IL} oder K_{S} umfasst, und einer durch den Rest R substituierten Cyanogruppe (R-CN).

6. Verfahren zur Herstellung eines Elektrolytsystems gemäß Anspruch 1,
mit einer Salzaustauschreaktion in Wasser zwischen einem Alkalisalz des Tetrazolidanions A und einem Chloridsalz, welches ein erfindungsgemäßes Kation K_{IL} oder K_{S} umfasst.

## Claims

1. Electrolyte system for use in an electrochemical component,
comprising at least one tetrazolide anion A with a substituent R in the 5-position according to the following formula 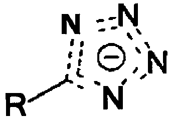 and at least one cation,
***characterised in that***
the substituent R is selected from the group:
a) R = fluorine group (-F),
b) R = trifluoromethyl group (-CF₃),
c) R = alkyl radical (R_{f}), which is completely or partly fluorinated with C1 to C8, in which the alkyl radical may be linear or branched,
d) R = a sulfonyl group substituted by an electron-withdrawing substituent,
e) R = a carboxyl group substituted by an electron-withdrawing substituent,
f) R = an ether group substituted by an electron-withdrawing substituent,
g) R = cyanosulfonyl group (-SO₂CN),
and
that the electrolyte system comprises at least one cation K_{IL} from the group:
a) K_{IL} = ammonium cations with various radicals (NRR¹R²R³),
with R, R¹, R², R³ = -(CH₂)ₙ-CH₃ and n = 0, 1, 2, 3,4, 5, 6, 7,
in which the radicals R, R¹, R², R³ may be the same or different,
b) K_{IL} = imidazolium cations with various radicals in the 1-, 2-, 3-, 4- and 5-position (RR¹R²R³R⁴lm), with R, R¹, R², R³, R⁴ = H, -(CH₂)ₙ-CH₃ and n = 0, 1, 2, 3, 4, 5, 6, 7, in which the radicals R, R¹, R², R³, R⁴ may be the same or different,
c) K_{IL} = pyrrolidinium cations with various radicals in the 1-position (RR¹Py),
with R, R¹ = -(CH₂)ₙ-CH₃ and n = 0, 1, 2, 3, 4, 5, 6, 7, in which the radicals R, R¹ may be the same or different,
d) K_{IL} = piperidinium cations with various radicals in the 1-position (RR¹Pip),
with R, R¹ = -(CH₂)ₙ-CH₃ and n = 0, 1, 2, 3, 4, 5, 6, 7, in which the radicals R and R¹ may be the same or different,
e) K_{IL} = pyridinium cations with various radicals in the 1-position (RPyri),
with R = -(CH₂)ₙ-CH₃ and n = 0,1, 2, 3, 4, 5, 6, 7,
f) K_{IL} = pyrazolium cations with various radicals in the 1-, 2-, 3-, 4- and 5-position (RR¹R²R³R⁴Pyr), with R, R¹, R², R³, R⁴ = H, -(CH₂)ₙ-CH₃ and n = 0,1, 2, 3, 4, 5, 6, 7, in which the radicals R, R¹, R², R³, R⁴ may be the same or different,
g) K_{IL} = phosphonium cations with various radicals (PRR¹R²R³), with R, R¹, R², R³ = -(CH₂)ₙ-CH₃ and n = 0, 1, 2, 3, 4, 5, 6, 7, in which the radicals R, R¹, R², R³ may be the same or different,
h) K_{IL} = sulfonium cations with various radicals (SRR¹R²), with R, R¹, R² = -(CH₂)ₙ-CH₃ and n = 0, 1, 2, 3, 4, 5, 6, 7, in which the radicals R, R¹, R², R³ may be the same or different,
or at least one cation K_{S} from the group:
a) K_{S} = H⁺,
b) K_{S} = Li⁺,
c) K_{S} = Na⁺,
d) K_{S} = K⁺,
e) K_{S} = Mg²⁺,
f) K_{S} = Ca²⁺,
g) K_{S} = Ni²⁺,
h) K_{S} = Pb²⁺,
i) K_{S} = Fe^{2+/3+},
j) K_{S} = V^{2+/3+/4+/5+}.

2. Electrolyte system according to claim 1 with a tetrazolide anion A substituted in the 5-position with a substituent R, in which R is selected from the group:
R = fluorosulfonyl group (-SO₂F),
R = trifluoromethylsulfonyl group (-SO₂CF₃) or
R = sulfonyl group with a higher fluorinated akyl radical (-SO₂R_{f}) with R_{f} = -(CF₂)ₙCF₃ and with n = 1, 2, 3, 4, 5, 6 or 7.

3. Electrolyte system according to claim 1 with a tetrazolide anion A substituted in the 5-position with a substituent R = carboxyl group (-OCOR_{f}) with R_{f} = (CF₂)ₙCF₃ and with n = **1, 2, 3,** 4, 5, 6 or 7.

4. Electrolyte system according to claim 1 with a tetrazolide anion A substituted in the 5-position with a substituent **R** = alkoxy group **(-OR_{f}) w**ith **R_{f}** = (CF₂)ₙCF₃ and with n = 1, 2, 3, 4, 5, 6, or 7.

5. Method for producing an electrolyte system according to claim 1,
with a [2+3] cycloaddition reaction in organic solvents between an ionic acid, which comprises a cation K_{IL} or K_{S} according to the invention and a cyano group (R-CN) substituted by the radical R.

6. Method for producing an electrolyte system according to claim 1,
with a salt exchange reaction in water between an alkali salt of the tetrazolide anion A and a chloride salt, which comprises a cation K_{IL} or K_{S} according to the invention.

## Revendications

1. Système d'électrolyte à utiliser dans un composant électrochimique,
comprenant au moins un anion A tétrazolide ayant un substituant R en la position 5, selon la formule suivante 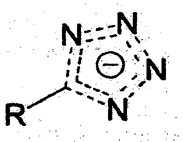 ainsi qu'au moins un cation,
**caractérisé**
**en ce que** le substituant R est choisi dans le groupe :
a) R = groupe fluor (-F),
b) R = groupe trifluorométhyle (-CF₃),
c) R = radical alcoyle (R_{f}) fluoré en tout ou partie en C1 à C8, le radical alcoyle pouvant être linéaire ou ramifié,
d) R = un groupe sulfonyle substitué par un substituant attirant les électrons,
e) R = un groupe carboxyle substitué par un substituant attirant les électrons,
f) R = un groupe éther substitué par un substituant attirant les électrons,
g) R = groupe cyanosulfonyle (-SO₂CN)
et
**en ce que** le système d'électrolyte comprend au moins un cation K_{IL} du groupe :
a) K_{IL} = cations ammonium ayant des radicaux (NRR¹R²R³) différents,
avec R, R¹, R², R³ = -(CH₂)ₙ-CH₃ et n = 0, 1, 2, 3, 4, 5, 6, 7,
les radicaux R, R¹, R², R³ pouvant être différents ou également les mêmes,
b) K_{IL} = cations imidazolium ayant des radicaux différents en la position 1, 2, 3, 4 et (RR¹R²R³R⁴=H, -(CH₂)ₙ-CH₃ et n = 0, 1, 2, 3, 4, 5, 6, 7, les radicaux R, R¹, R², R³, R⁴ pouvant être différents ou aussi les mêmes,
c) K_{IL} = cations pyrrolidinium ayant des radicaux différents en position 1 (RR¹Py),
avec R, R¹ = -(CH₂)ₙ-CH₃ et n = 0, 1, 2, 3, 4, 5, 6, 7, les radicaux R, R¹ pouvant être différents ou aussi les mêmes,
d) K_{IL} = cations pipéridinium ayant des radicaux différents en position 1 (RR¹Pip),
avec R, R¹ = -(CH₂)ₙ-CH₃ et n = 0, 1, 2, 3, 4, 5, 6, 7, les radicaux R et R¹ pouvant être différents ou aussi les mêmes,
e) K_{IL} = cations pyrydinium ayant des radicaux différents en position 1 (RPyri),
avec R = -(CH₂)ₙ-CH₃ et n = 0, 1, 2, 3, 4, 5, 6, 7,
f) K_{IL} = cations pyrazolium ayant des radicaux différents en position 1, 2, 3, 4 et 5 (RR¹R²R³R⁴Pyr),
avec R, R¹, R², R³, R⁴ = H, -(CH₂)ₙ-CH₃ et n = 0, 1, 2, 3, 4, 5, 6, 7, les radicaux R, R¹, R², R³, R⁴ pouvant être différents ou également les mêmes,
g) K_{IL} = cations phosphonium ayant des radicaux différents (PRR¹R²R³) avec R, R¹, R², R³ = (CH₂)ₙ-CH₃ et n = 0, 1, 2, 3, 4, 5, 6, 7, les radicaux R, R¹, R², R³ pouvant être différents ou aussi les mêmes,
h) K_{IL} = cations sulfonium ayant des radicaux (SRR¹R²) différents avec R, R¹, R² = -(CH₂)ₙ-CH₃ et n = 0, 1, 2, 3, 4, 5, 6, 7, les radicaux R, R¹, R², R³ pouvant être différents ou aussi les mêmes,
ou au moins un cation Kₛ du groupe :
a) Kₛ = H⁺,
b) Kₛ = Li⁺,
c) Kₛ = Na⁼,
d) Kₛ = K⁺,
e) Kₛ = Mg²⁺,
f) Kₛ = Ca²⁺,
g) Kₛ = Ni²⁺,
h) Kₛ = Pb²⁺,
i) Kₛ = Fe^{2+/3+}
j) Kₛ = V^{2+/3+/4+/5+.}

2. Système d'électrolyte suivant la revendication 1, ayant un anion A tétrazolide substitué en position 5 par un substituant R, R étant choisi dans le groupe :
R = groupe fluorosulfonyle (-SO₂F),
R = groupe trifluorométhylsulfonyle (-SO₂CF₃) ou
R = groupe sulfonyle ayant un radical alcoyle supérieur fluoré (-SO₂R_{f}) avec R_{f} = -(CF₂)ₙCF₃ et n =1, 2, 3, 4, 5, 6 ou 7.

3. Système d'électrolyte suivant la revendication 1, ayant un anion A tétrazolide substitué en position 5 par un substituant R = groupe carboxyle (-OCOR_{f}) avec R_{f} = (CF₂)ₙCF₃ et avec n = 1, 2, 3, 4, 4, 5, 6 ou 7.

4. Système d'électrolyte suivant la revendication 1, ayant un anion A tétrazolide substitué en position 5 par un substituant R = groupe alcoxy (-OR_{f}) avec R_{f} = (CF₂)ₙCF₃ et avec n = 1, 2, 3, 4, 5, 6, ou 7.

5. Procédé de préparation d'un système d'électrolyte suivant la revendication 1,
par une réaction de cycloaddition [2+3] dans des solvants organiques entre un azide ionique, qui comprend un cation K_{IL} ou K_{S} suivant l'invention et un groupe cyano (R-CN) substitué par le radical R.

6. Procédé de préparation d'un système d'électrolyte suivant la revendication 1,
comprenant une réaction d'échange de sel entre un sel de métal alcalin de l'anion A tétrazolide et un chlorure, qui comprend un cation K_{IL} ou K_{S} suivant l'invention.
